# EUROPEAN PATENT APPLICATION

(11) **EP 3 919 489 A1**
(43) Date of publication of application: **08.12.2021**
(21) Application number: 20749262.0
(22) Date of filing: 17.01.2020
(51) Int. Cl.: C07D 417/14, C07D 451/06, C07D 471/04, A61K 31/46, A61P 3/00

(54) **AROMATIC RING OR HETEROAROMATIC RING COMPOUNDS, PREPARATION METHOD THEREFOR AND MEDICAL USE THEREOF**

(30) Priority: 31.01.2019 CN 201910096771
(71) Applicant: The National Institutes of Pharmaceutical R&D Co., Ltd, Changping District Beijing 102206 (CN)
(72) Inventor: YIN, Huijun, Beijing 102206 (CN); YAN, Xu, Beijing 102206 (CN); ZONG, Libin, Beijing 102206 (CN); SHI, Jianxin, Beijing 102206 (CN); LIU, Chunyan, Beijing 102206 (CN); ZHANG, Shouliang, Beijing 102206 (CN); LU, Jiawei, Beijing 102206 (CN); LI, Hao, Beijing 102206 (CN)
(74) Representative: EP&C
(86) International application number: PCT/CN2020/072763
(87) International publication number: WO 2020/156241

(57) **Abstract**

The present invention relates to aromatic ring or heteroaromatic ring compounds, a preparation method therefor and a medical use thereof. Particularly, the present invention relates to a compound as shown in general formula (I) and a preparation method therefor, a pharmaceutical composition comprising the compound and a use thereof as an agonist for a farnesoid X receptor (FXR). The compound and the pharmaceutical composition comprising the compound can be used for treating and/or preventing FXR activity-related diseases, for example, cholestatic symptoms, diabetes and complications thereof, nonalcoholic fatty liver disease (NAFLD), nonalcoholic steatosis heptitis (NASH), obesity or metabolic syndrome (diseases associated with dyslipidemia, diabetes, abnormally high body weight index), cardiovascular diseases and so on. The definition of each substituent in the general formula (I) is the same as that in the description.

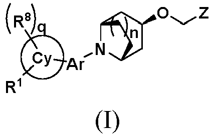

## Description

### FIELD OF THE INVENTION

The present invention belongs to the field of medical technology, and specifically relates to an aromatic ring or heteroaromatic ring compound, a method for preparing the same and a pharmaceutical composition comprising the same, as well as a use thereof for regulating Farnesoid X receptor (FXR) activity and treating and/or preventing a disease related to FXR activity.

### BACKGROUND OF THE INVENTION

As a nuclear receptor activated by bile acid, farnesoid X receptor (FXR) can regulate the expression of a variety of metabolism-related genes directly or through the orphan nuclear receptor small heterodimer partner (SHP). FXR was discovered in 1995, and its name was derived from the fact that the receptor can be activated by superphysiological level of farnesol. Recent studies have found that bile acids (BAs) are the most important physiological ligand of FXR (Drug Discovery Today, 2012, 17, 988).

Abnormal level of bile acid is related to liver inflammation and fibrosis. The accumulation of bile acid is a more important pathogenic factor for non-alcoholic fatty liver compared with triglyceride. The activation of FXR can achieve the net effect of reducing the accumulation of bile acid in the liver by reducing the synthesis and uptake of bile acid in the liver and increasing the efflux of bile acid. FXR can up-regulate SHP, and SHP can inhibit the expression of CYP7A1, an important enzyme for bile acid synthesis, thereby inhibiting bile acid synthesis (Pharmacol. Ther. 2010, 126, 228-243). Moreover, FXR can induce FGF15/19 to activate FGFR4, thereby initiating the JNK pathway to inhibit the expression of CYP7A1. FXR inhibits NTCP, reduces the level of bile acid in hepatocytes, stimulates the secretion of bile acid in the hepatocyte tubule membrane, and inhibits the reuptake of bile acid from the portal vein by up-regulating BSEP and MRP2. FXR can regulate the bile acid transport through OSTα/β, promote the excretion of bile acid to the circulatory system and elimination by the kidneys.

FXR can improve insulin resistance. FXR knockout mice showed impaired glucose metabolism clearance, indicating that the mice have peripheral insulin resistance. The improvement effect of FXR activation on insulin resistance may be related to the following mechanisms: FXR can reduce the accumulation of lipid in peripheral tissues (such as muscle cells), thereby reducing lipid-related toxicity, especially for obese patients caused by diet (Acta. Pharmacol. Sin. 2015, 36, 44-50); the activation of FXR in the small intestine can promote the release of FGF19 to the portal vein, FGF19 has a certain insulin sensitization effect, it is also reported that FGF 19 has the effect of reducing body weight, and FGF19 transgenic mice showed strong resistance to diet-induced obesity; FXR can reduce gluconeogenesis and glycogen output, feeding mice with cholic acid can inhibit the expression of gluconeogenesis-related enzyme (such as PEPCK, G6P) genes, but this has no effect on SHP knockout mice; FXR agonists can reduce PEPCK and G6P in mice, and reduce glycogen output.

FXR can reduce the production of triglycerides and fatty acids and promote their metabolism through a variety of ways. FXR can inhibit the expression of SRFBP1c, inhibit the synthesis and secretion of triglycerides and fatty acids, promote the expression of VLDLR, improve the clearance of VLDL and chylomicrons, inhibit the expression of APOC and MTP, inhibit the assembly of VLDL, induce PPARα, promote β-oxidation of fatty acids, induce lipoprotein lipase, and enhance the metabolism of lipoproteins and free fatty acids. FXR promotes the uptake of HDL and the reverse transport of cholesterol by positively regulating SRBI, CEH, SCP2 and the like, and achieves a cholesterol reducing effect by inhibiting the expression and activity of PCSK9 and enhancing the clearance of LDLR and LDL (Curr. Opin. Lipidol. 2016, 27, 295-301).

FXR can inhibit inflammation by down-regulating the expression of a variety of inflammation-related genes. FXR is closely related to inflammation. FXR knockout mice have higher level of pro-inflammatory cytokines and pro-fibrocytokines, including TNFα, ICAM-1, α-SMA, TIMP-1, TGFβ and the like. The inhibition of inflammation and fibrosis by FXR may be related to the following mechanisms: the main inflammation inhibitory mechanism activated by FXR is to antagonize the NFκB signaling pathway; FXR activation can improve bile duct obstruction, intestinal flora overgrowth, mucosal damage, intestinal bacterial translocation and the like; FXR can induce the inhibitory factor of SOCS3, thereby inhibiting the STAT3 signaling pathway; FXR activation can increase MicroRNA mir29a, which can regulate the expression of multiple extracellular matrix proteins. In addition, other studies have shown that FXR can promote liver tissue regeneration and inhibit the occurrence of hepatocellular tumors. Therefore, FXR agonists can be used for the prevention and treatment of lipid (especially triglyceride) accumulation and diseases and disorders caused by triglyceride accumulation and chronic steatosis and fibrosis, such as non-alcoholic fatty liver disease (NAFLD) or non-alcoholic steatohepatitis (NASH) (Adv. Ther. 2016, 33, 291-319; Drug Discov. Today, 2012, 17, 988-97).

At present, FXR agonist has become one of the hottest topics in global innovative drug research and development. Among the FXR agonists, the steroidal FXR agonist obeticholic acid has been successfully approved for use in primary biliary cirrhosis (PBC), and has shown good efficacy in clinical studies (F1INT studies) for the treatment of NASH, further demonstrating the rationality of FXR agonist for the treatment of PBC and NASH. However, as a steroidal drug, this drug has poor selectivity. In particular, it has certain effect on TGR5, and can easily cause side effects such as severe itching and hyperlipidemia. Moreover, obeticholic acid has strong hepato-intestinal circulation, which leads to a high accumulation of drug in the body and causes safety risks. Therefore, non-steroidal FXR agonists with higher selectivity and better pharmacokinetic properties have attracted more attention (Lancet, 2015, 385, 956-65; N. Engl. J. Med. 2016, 375, 631-643).

The non-steroidal FXR agonist studied earlier is GW-4604. This compound has strong *in vitro* activity with poor pharmacokinetic properties, and has safety risks due to the diarylethene structure. At present, the most concerned drug having the fastest research and development progress is PX-104. This drug has high FXR agonistic activity and pharmacokinetic properties, and has shown a significant reduction in liver fat accumulation and damage in preclinical pharmacodynamic models. It has now entered the phase II clinical study for treating NASH (J. Pharmacol. Exp. Ther. 2012, 343, 556-567; J. Med. Chem. 2014, 57, 8035-8055). Nevertheless, the *in vitro* activity of PX-104 still has room for further improvement, and the clinical dose thereof is relatively high. Therefore, there is a continuing need for new or improved drugs that stimulate FXR for the development of new and more effective drugs to treat NASH, NAFLD, PBC or other FXR-related diseases.

### SUMMARY OF THE INVENTION

After deep research, the inventors have designed and synthesized a series of compounds containing an aromatic ring or heteroaromatic ring moiety substituted by a bridged ring, and screened for FXR activity. Research results show that these compounds have outstanding FXR agonistic activity, and can be developed as drugs for treating diseases related to FXR activity.

Thus, an object of the present invention is to provide a compound of formula (I) or a mesomer, racemate, enantiomer, diastereomer thereof, or mixture thereof, or a prodrug thereof, or a pharmaceutically acceptable salt thereof, 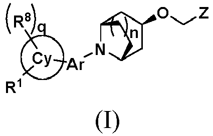 wherein:
Z is selected from the group consisting of 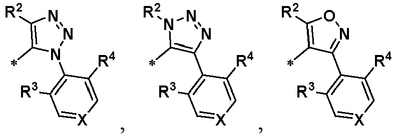 and 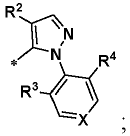
wherein X is CH, CF, N or NO;
R² is selected from the group consisting of hydrogen, halogen, alkyl and cycloalkyl, wherein the alkyl and the cycloalkyl are each optionally further substituted by one or more substituent(s) selected from the group consisting of halogen, hydroxy, alkyl and alkoxy;
R³ and R⁴ are each independently selected from the group consisting of hydrogen, halogen, alkyl and alkoxy, wherein the alkyl and the alkoxy are each optionally further substituted by one or more halogen;
Ar is a 5-membered or 6-membered aryl or a heteroaryl;
Cy is an aryl or a heteroaryl;
R¹ is selected from the group consisting of -(CH₂)ₘ-R⁵ and -O(CH₂)ₘ-R⁵, wherein the -(CH₂)ₘ- and -O(CH₂)ₘ- are each optionally further substituted by one or more substituent(s) selected from the group consisting of halogen, cyano, hydroxy, oxo, alkyl, haloalkyl, alkoxy, haloalkoxy, CO₂H and SO₃H;
R⁵ is selected from the group consisting of hydrogen, halogen, cyano, nitro, alkyl, cycloalkyl, heterocyclyl, aryl, heteroaryl, OR⁶, NR⁶R⁷, -CO₂R⁶, -C(O)R⁶, -C(O)NR⁶R⁷, -N(R⁶)C(O)R⁷, -C(O)NR⁶SO₂R⁷, -S(O)ₚR⁶, -S(O)ₚNR⁶R⁷, -N(R⁶)S(O)ₚR⁷ and -S(O)ₚNR⁶COR⁷, wherein the alkyl, cycloalkyl, heterocyclyl, aryl and heteroaryl are each optionally further substituted by one or more substituent(s) selected from the group consisting of halogen, amino, nitro, cyano, hydroxy, thiol, carboxyl, ester group, oxo, alkyl, haloalkyl, alkoxy, haloalkoxy, alkenyl, alkynyl, cycloalkyl, halocycloalkyl, heterocyclyl, aryl and heteroaryl;
R⁶ and R⁷ are each independently selected from the group consisting of hydrogen, halogen, hydroxy, alkyl, cycloalkyl, heterocyclyl, aryl and heteroaryl, wherein the alkyl, cycloalkyl, heterocyclyl, aryl and heteroaryl are each optionally further substituted by one or more substituent(s) selected from the group consisting of halogen, amino, nitro, cyano, hydroxy, thiol, carboxyl, ester group, oxo, alkyl, alkoxy, cycloalkyl, heterocyclyl, aryl and heteroaryl;
or, R⁶ and R⁷ together with the nitrogen atom to which they are attached form a nitrogen-containing heterocyclyl, wherein the nitrogen-containing heterocyclyl is optionally further substituted by one or more substituent(s) selected from the group consisting of halogen, amino, nitro, cyano, oxo, hydroxy, thiol, carboxyl, ester group, alkyl, alkoxy, cycloalkyl, heterocyclyl, aryl and heteroaryl;
each R⁸ can be identical or different and are each independently selected from the group consisting of halogen, amino, nitro, cyano, hydroxy, thiol, carboxyl, ester group, alkyl, haloalkyl, alkoxy, haloalkoxy, alkenyl, alkynyl, cycloalkyl, halocycloalkyl, heterocyclyl, aryl and heteroaryl;
n is 0, 1 or 2;
m is an integer from 0 to 6;
p is 0, 1 or 2;
q is an integer from 0 to 4;
with the proviso that Ar is not 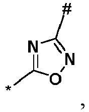 wherein ^{∗} represents the site connected to Cy, and # represents the site connected to the N of the bridged ring.

In a preferred embodiment of the present invention, the compound of formula (I) or the mesomer, racemate, enantiomer, diastereomer thereof, or mixture thereof, or the prodrug thereof, or the pharmaceutically acceptable salt thereof according to the present invention is a compound of formula (II) or a mesomer, racemate, enantiomer, diastereomer thereof, or mixture thereof, or a prodrug thereof, or a pharmaceutically acceptable salt thereof, 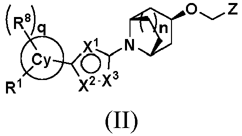 wherein,
X¹, X² and X³ are each independently selected from the group consisting of C, N, O and S, and preferably N and O;
Z, n, Cy, R¹, R⁸ and q are as defined in formula (I).

In another preferred embodiment of the present invention, the compound of formula (I) or the mesomer, racemate, enantiomer, diastereomer thereof, or mixture thereof, or the prodrug thereof, or the pharmaceutically acceptable salt thereof according to the present invention,
wherein, Ar is selected from the group consisting of oxazole, isoxazole, thiazole, isothiazole, imidazole, triazole, 1,2,3-oxadiazole, 1,2,4-oxadiazole, 1,3,4-oxadiazole, 1,2,3-thiadiazole, 1,2,4-thiadiazole and 1,3,4-thiadiazole.

In another preferred embodiment of the present invention, the compound of formula (I) or the mesomer, racemate, enantiomer, diastereomer thereof, or mixture thereof, or the prodrug thereof, or the pharmaceutically acceptable salt thereof according to the present invention, wherein,
Z is 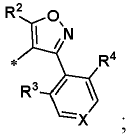
X is selected from the group consisting of CH and N;
R² is selected from the group consisting of hydrogen, halogen, alkyl and cycloalkyl, preferably C₁-C₆ alkyl and C₃-C₆ cycloalkyl, and more preferably cyclopropyl, wherein the alkyl and cycloalkyl are each optionally further substituted by one or more substituent(s) selected from the group consisting of halogen, hydroxy, alkyl and alkoxy;
R³ and R⁴ are each independently selected from the group consisting of hydrogen, halogen, alkyl, haloalkyl, alkoxy and haloalkoxy, and preferably hydrogen, halogen, C₁-C₆ haloalkyl and C₁-C₆ haloalkoxy.

In another preferred embodiment of the present invention, the compound of formula (I) or the mesomer, racemate, enantiomer, diastereomer thereof, or mixture thereof, or the prodrug thereof, or the pharmaceutically acceptable salt thereof according to the present invention,
wherein, n is 1.

In another preferred embodiment of the present invention, the compound of formula (I) or the mesomer, racemate, enantiomer, diastereomer thereof, or mixture thereof, or the prodrug thereof, or the pharmaceutically acceptable salt thereof according to the present invention,
wherein,
Cy is a C₅-C₆ aryl or a 5- to 6-membered heteroaryl, and preferably phenyl, pyridyl, pyrimidinyl, pyrazinyl, thiazolyl, furyl, imidazolyl or pyrazolyl.

In another preferred embodiment of the present invention, the compound of formula (I) or the mesomer, racemate, enantiomer, diastereomer thereof, or mixture thereof, or the prodrug thereof, or the pharmaceutically acceptable salt thereof according to the present invention,
wherein,
R¹ is selected from the group consisting of -(CH₂)ₘ-R⁵ and -O(CH₂)ₘ-R⁵;
R⁵ is selected from the group consisting of hydrogen, halogen, alkyl, OR⁶, NR⁶R⁷, -CO₂R⁶, -C(O)R⁶, -C(O)NR⁶R⁷, -N(R⁶)C(O)R⁷, -C(O)NR⁶SO₂R⁷, -S(O)ₚR⁶, -S(O)ₚ NR⁶R⁷, -N(R⁶)S(O)ₚR⁷ and -S(O)ₚNR⁶COR⁷, preferably -C(O)R⁶, -C(O)NR⁶R⁷, -S(O)ₚR⁶ and -S(O)ₚNR⁶R⁷, more preferably -C(O)R⁶ and -S(O)ₚNR⁶R⁷, and even more preferably -COOH;
R⁶ and R⁷ are each independently selected from the group consisting of hydrogen, halogen, hydroxy, alkyl, cycloalkyl, heterocyclyl, aryl and heteroaryl, wherein the alkyl, cycloalkyl, heterocyclyl, aryl and heteroaryl are each optionally further substituted by one or more substituent(s) selected from the group consisting of halogen, amino, nitro, cyano, hydroxy, thiol, carboxyl, ester group, oxo, alkyl, alkoxy, cycloalkyl, heterocyclyl, aryl and heteroaryl;
or, R⁶ and R⁷ together with the nitrogen atom to which they are attached form a nitrogen-containing heterocyclyl, wherein the nitrogen-containing heterocyclyl is optionally further substituted by one or more substituent(s) selected from the group consisting of halogen, amino, nitro, cyano, oxo, hydroxy, thiol and carboxyl;
m is an integer from 0 to 6, preferably 0, 1 or 2, and more preferably 0.

In another preferred embodiment of the present invention, the compound of formula (I) or the mesomer, racemate, enantiomer, diastereomer thereof, or mixture thereof, or the prodrug thereof, or the pharmaceutically acceptable salt thereof according to the present invention,
wherein,
each R⁸ can be identical or different and are each independently selected from the group consisting of halogen, alkyl, haloalkyl, alkoxy and haloalkoxy;
q is an integer from 0 to 4; and preferably q is 0 or 1.

Typical compounds of the present invention include, but are not limited to:
4-(5-((1R,3r,5S)-3-((5-cyclopropyl-3-(2,6-dichlorophenyl)-isoxazol-4-yl)methoxy) -8-azabicyclo[3.2.1]octan-8-yl)-1,2,4-oxadiazol-3-yl)benzoic acid;
2-chloro-4-(5-((1R,3r,5S)-3-((5-cyclopropyl-3-(2,6-dichlorophenyl)isoxazol-4-yl) methoxy)-8-azabicyclo[3.2.1]octan-8-yl)-1,2,4-oxadiazol-3-yl)benzoic acid;
6-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)pyridine-2-carboxylic acid;
5-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)thiophene-2-carboxylic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)-3-methylbenzoic acid;
3-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)benzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)-2-methylbenzoic acid;
4-(5-((1R,3r,5S)-3-((5-cyclopropyl-3-(2,6-dichlorophenyl)isoxazol-4-yl)methoxy)-8-azabicyclo[3.2.1]octan-8-yl)-1,3,4-oxadiazol-2-yl)benzoic acid;
2-chloro-4-(5-((1R,3r,5S)-3-((5-cyclopropyl-3-(2,6-dichlorophenyl)isoxazol-4-yl) methoxy)-8-azabicyclo[3.2.1]octan-8-yl)-1,3,4-oxadiazol-2-yl)benzoic acid;
6-(5-((1R,3r,5S)-3-((5-cyclopropyl-3-(2,6-dichlorophenyl)isoxazol-4-yl)methoxy)-8-azabicyclo[3.2.1]octan-8-yl)-1,3,4-oxadiazol-2-yl)pyridine-2-carboxylic acid;
5-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)thiophene-2-carboxylic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-l,3,4-oxadiazol-2-yl)-2-fluorobenzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-2-methoxybenzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-3-methylbenzoic acid;
3-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)benzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-3-fluorobenzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-2-methylbenzoic acid;
5-(5-((1R,3r,5S)-3-((5-cyclopropyl-3-(2,6-dichlorophenyl)isoxazol-4-yl)methoxy)-8-azabicyclo[3.2.1]octan-8-yl)-1,3,4-oxadiazol-2-yl)pyridine-carboxylic acid;
6-(5-((1R,3r,5S)-3-((5-cyclopropyl-3-(2,6-dichlorophenyl)isoxazol-4-yl)methoxy)-8-azabicyclo[3.2.1]octan-8-yl)-1,3,4-oxadiazol-2-yl)nicotinic acid;
5-(5-((1R,3r,5S)-(3-((5-cyclopropyl-3-(2,6-dichlorophenyl)isoxazol-4-yl)methoxy) -8-azabicyclo[3.2.1]octan-8-yl)-l,3,4-oxadiazol-2-yl)thiophene-3-carboxylic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)thiophene-2-carboxylic acid;
4-(5-((1R,3r,5S)-3-((5-cyclopropyl-3-(2-(trifluoromethoxy)phenyl)isoxazol-4-yl)m ethoxy)-8-azabicyclo[3.2.1]octan-8-yl)-1,3,4-oxadiazol-2-yl)benzoic acid;
2-chloro-4-(5-((1R,3r,5S)-3-((5-cyclopropyl-3-(2-(trifluoromethoxy)phenyl)isoxaz ol-4-yl)methoxy)-8-azabicyclo[3.2.1]octan-8-yl)-1,3,4-oxadiazol-2-yl)benzoic acid;
6-(5-((1R,3r,5S)-3-((5-cyclopropyl-3-(2-(trifluoromethoxy)phenyl)isoxazol-4-yl)m ethoxy)-8-azabicyclo[3.2.1]octan-8-yl)-1,3,4-oxadiazol-2-yl)pyridine-2-carboxylic acid;
5-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)thiophene-2-carboxylic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)-2-fluorobenzoic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)-3-methylbenzoic acid;
3-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)benzoic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)-3-fluorobenzoic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)-2-methylbenzoic acid;
5-(5-((1R,3r,5S)-3-((5-cyclopropyl-3-(2-(trifluoromethoxy)phenyl)isoxazol-4-yl)m ethoxy)-8-azabicyclo[3.2.1]octan-8-yl)-1,3,4-oxadiazol-2-yl)pyridine-carboxylic acid;
6-(5-((1R,3r,5S)-3-((5-cyclopropyl-3-(2-(trifluoromethoxy)phenyl)isoxazol-4-yl)m ethoxy)-8-azabicyclo[3.2.1]octan-8-yl)-1,3,4-oxadiazol-2-yl)nicotinic acid;
5-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)thiophene-3-carboxylic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)thiophene-2-carboxylic acid;
or a mesomer, racemate, enantiomer, diastereomer thereof, or mixture thereof, or a prodrug thereof, or a pharmaceutically acceptable salt thereof.

The present invention further provides a method for preparing the compound of formula (I) or the mesomer, racemate, enantiomer, diastereomer thereof, or mixture thereof, or the prodrug thereof, or the pharmaceutically acceptable salt thereof according to the present invention, comprising the following step of: 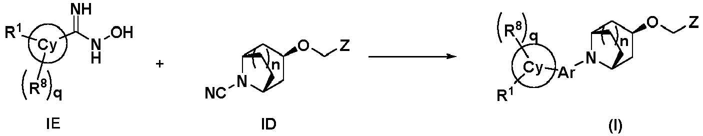 subjecting compound IE and compound ID to a cyclization reaction in the presence of a condensing agent to obtain the compound of formula (I), wherein the condensing agent is preferably zinc chloride;
wherein Ar is 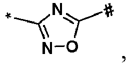 wherein ^{∗} represents the site connected to Cy, and # represents the site connected to the N of the bridged ring,
Z, n, Cy, R¹, R⁸ and q are as defined in formula (I).

The present invention further provides a method for preparing the compound of formula (I) or the mesomer, racemate, enantiomer, diastereomer thereof, or mixture thereof, or the prodrug thereof, or the pharmaceutically acceptable salt thereof according to the present invention, comprising the following step of: 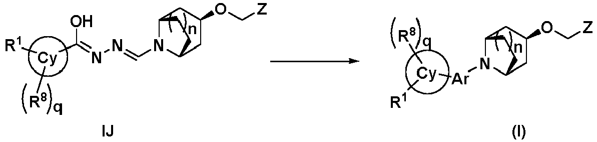 subjecting compound IJ to a cyclization reaction in the presence of an oxidizing agent to obtain the compound of formula (I), wherein the oxidizing agent is preferably iodobenzene diacetate;
when Ar is 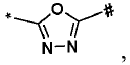 wherein ^{∗} represents the site connected to Cy, and # represents the site connected to the N of the bridged ring,
Z, n, Cy, R¹, R⁸ and q are as defined in formula (I).

In another aspect, the present invention provides a pharmaceutical composition comprising the compound of formula (I) or the mesomer, racemate, enantiomer, diastereomer thereof, or mixture thereof, or the prodrug thereof, or the pharmaceutically acceptable salt thereof according to the present invention, and a pharmaceutically acceptable carrier.

The present invention further provides a use of the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention in the preparation of a FXR agonist.

The present invention further provides a use of the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention in the preparation of a medicament for preventing and/or treating a disease related to FXR activity.

The present invention provides the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention, for use as a FXR agonist.

The present invention further provides the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention, for use as a medicament, wherein the medicament is used for preventing and/or treating a disease related to FXR activity.

The present invention further provides a method for preventing and/or treating a disease related to FXR activity, comprising a step of administering a preventively or therapeutically effective dose of the compound of formula (I) or the mesomer, racemate, enantiomer, diastereomer thereof, or mixture thereof, or the prodrug thereof, or the pharmaceutically acceptable salt thereof, or the pharmaceutical composition comprising the same according to the present invention to a subject in need thereof.

In a preferred embodiment of the present invention, the disease related to FXR activity according to the present invention can be: chronic intrahepatic cholestasis or extrahepatic cholestasis, or liver fibrosis caused by chronic cholestasis or acute intrahepatic cholestasis; and/or liver obstructive or chronic inflammation; and/or liver cirrhosis; and/or hepatic steatosis and related syndromes, cholestasis or fibrosis associated with alcohol-induced cirrhosis or viral hepatitis; and/or liver failure or liver ischemia after liver resection; and/or chemotherapy related to steatohepatitis; and/or acute liver failure; and/or inflammatory bowel disease; and/or lipid and lipid protein disorders; and/or diabetes and clinical complications of diabetes, including diabetic nephropathy, diabetic neuropathy, diabetic retinopathy and other clinical manifestations; and/or lipids, especially triglyceride, accumulation, and diseases and disorders caused by chronic fat and fibrosis due to triglyceride accumulation, such as non-alcoholic fatty liver or non-alcoholic steatohepatitis; and/or obesity or metabolic syndrome, such as dyslipidemia, diabetes, and comorbidities with abnormally high body mass index; and/or acute myocardial infarction, acute stroke or thrombosis as the end point of chronic obstructive atherosclerosis; non-malignant hyperproliferative diseases and malignant hyperproliferative diseases, especially hepatocellular carcinoma, colonic adenoma and polyposis, colon adenocarcinoma, breast cancer, pancreatic cancer, Bart's esophagus cancer and other forms of gastrointestinal and liver neoplastic diseases.

The compound of formula (I) of the present invention can form a pharmaceutically acceptable acid addition salt with an acid according to the conventional methods in the art. The acid includes an inorganic acid or an organic acid, and particularly preferably hydrochloric acid, hydrobromic acid, sulfuric acid, phosphoric acid, methanesulfonic acid, ethanesulfonic acid, *p*-toluenesulfonic acid, benzenesulfonic acid, naphthalene disulfonic acid, acetic acid, propionic acid, lactic acid, trifluoroacetic acid, maleic acid, citric acid, fumaric acid, oxalic acid, tartaric acid, benzoic acid and the like.

The compound of formula (I) of the present invention can form a pharmaceutically acceptable base addition salt with a base according to the conventional methods in the art. The base includes an inorganic base or an organic base. Acceptable organic bases include diethanolamine, ethanolamine, N-methylglucamine, triethanolamine, tromethamine and the like, and acceptable inorganic bases include aluminum hydroxide, calcium hydroxide, potassium hydroxide, sodium carbonate, sodium hydroxide and the like.

Furthermore, the present invention also comprises the prodrugs of the compound of formula (I) of the present invention. The prodrugs of the present invention are derivatives of the compound of formula (I), which may have weak or even no activity *per se,* but can be converted to the corresponding biologically active forms under physiological conditions (for example by metabolism, solvolysis or other ways) upon administration.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such composition can contain one or more ingredients selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the preparation of tablets. These excipients may be inert excipients, such as calcium carbonate, sodium carbonate, lactose, calcium phosphate or sodium phosphate; granulating and disintegrating agents, such as microcrystalline cellulose, cross-linked sodium carboxylmethyl cellulose, corn starch or alginic acid; binders, such as starch, gelatin, polyvinylpyrrolidone or acacia; and lubricants, such as magnesium stearate, stearic acid or talc. The tablet may be uncoated or coated by means of known techniques, which can mask drug taste or delay the disintegration and absorption of the active ingredient in the gastrointestinal tract, thereby providing sustained release over an extended period. For example, a water-soluble taste masking material can be used, such as hydroxypropyl methylcellulose or hydroxypropyl cellulose, or an extended-release material can be used, such as ethyl cellulose, cellulose acetate butyrate.

An oral formulation can also be provided as hard gelatin capsules in which the active ingredient is mixed with an inert solid diluent, such as calcium carbonate, calcium phosphate or kaolin, or as soft gelatin capsules in which the active ingredient is mixed with a water-soluble carrier such as polyethylene glycol or an oil medium such as peanut oil, liquid paraffin or olive oil.

An aqueous suspension contains the active ingredient in admixture with an excipient suitable for the preparation of aqueous suspension. Such excipient is a suspending agent, such as sodium carboxylmethylcellulose, methylcellulose, hydroxypropylmethylcellulose, sodium alginate, polyvinylpyrrolidone and acacia; a dispersant or humectant, which can be a naturally occurring phosphatide such as lecithin, or a condensation product of an alkylene oxide with fatty acid such as polyoxyethylene stearate, or a condensation product of ethylene oxide with a long chain aliphatic alcohol such as heptadecaethyleneoxy cetanol, or a condensation product of ethylene oxide with part esters derived from fatty acids and hexitols such as polyoxyethylene sorbitol monooleate, or a condensation product of ethylene oxide with partial esters derived from fatty acids and hexitol anhydrides such as polyoxyethylene sorbitan monooleate. The aqueous suspension can also contain one or more preservatives, such as ethylparaben or *n*-propylparaben, one or more colorants, one or more flavoring agents, and one or more sweeteners such as sucrose, saccharin or aspartame.

An oil suspension can be formulated by suspending the active ingredient in a vegetable oil such as peanut oil, olive oil, sesame oil or coconut oil, or in a mineral oil such as liquid paraffin. The oil suspension can contain a thickener, such as beeswax, hard paraffin or cetyl alcohol. The above sweetener and flavoring agent can be added to provide a palatable formulation. These compositions can be preserved by adding an antioxidant, such as butylated hydroxyanisole or *α*-tocopherol.

The active ingredient in admixture with the dispersants or wetting agents, suspending agent or one or more preservatives can be prepared as a dispersible powder or granule suitable for the preparation of an aqueous suspension by adding water. Suitable dispersants or wetting agents and suspending agents are as described above. Additional excipients, such as sweetening agents, flavoring agents and coloring agents, can also be added. These compositions are preserved by adding an antioxidant such as ascorbic acid.

The pharmaceutical composition of the present invention can also be in the form of an oil-in-water emulsion. The oil phase can be a vegetable oil such as olive oil or peanut oil, or a mineral oil such as liquid paraffin or a mixture thereof. Suitable emulsifying agent can be naturally occurring phosphatides, such as soy bean lecithin, and esters or partial esters derived from fatty acids and hexitol anhydrides such as sorbitan monooleate, and condensation products of said partial esters with ethylene oxide such as polyoxyethylene sorbitol monooleate. The emulsion can also contain a sweetener, flavoring agent, preservative and antioxidant. Syrup and elixir can be formulated with a sweetener, such as glycerol, propylene glycol, sorbitol or sucrose. Such formulations can also contain a moderator, a preservative, a colorant and an antioxidant.

The pharmaceutical composition of the present invention can be in the form of a sterile injectable aqueous solution. The acceptable vehicles and solvents that can be employed include water, Ringer's solution and isotonic sodium chloride solution. The sterile injectable formulation can be a sterile injectable oil-in-water microemulsion in which the active ingredient is dissolved in the oil phase. For example, the active ingredient can be firstly dissolved in a mixture of soybean oil and lecithin, the oil solution is then introduced into a mixture of water and glycerol and processed to form a microemulsion. The injectable solution or microemulsion can be introduced into a patient's bloodstream by local bolus injection. Alternatively, it may be advantageous to administrate the solution or microemulsion in such a way as to maintain a constant circulating concentration of the compound of the present invention. In order to maintain such a constant concentration, a continuous intravenous delivery device can be utilized.

The pharmaceutical composition of the present invention can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent, such as a solution prepared in 1,3-butanediol. Moreover, sterile fixed oils can easily be used as a solvent or suspending medium. For this purpose, any blending fixed oils including synthetic mono- or di-glyceride can be employed. Moreover, fatty acids such as oleic acid can also be employed in the preparation of an injection.

The compound of the present invention can be administered in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug. Such materials include cocoa butter, glycerin gelatin, hydrogenated vegetable oils, mixtures of polyethylene glycols with various molecular weights and fatty acid esters of polyethylene glycols.

It is well known to those skilled in the art that the dosage of a drug depends on a variety of factors including, but not limited to the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of the present invention or the type of pharmaceutically acceptable salt thereof can be verified according to the traditional therapeutic regimens.

The present invention may contain a composition comprising the compound of formula (I) or the pharmaceutically acceptable salt, hydrate or solvate as an active ingredient, and a pharmaceutically acceptable carrier or excipient, which is formulated into a clinically acceptable formulation. The derivatives of the present invention can be used in combination with other active ingredients as long as they do not cause other adverse effects such as allergic reactions and the like. The compound of the present invention can be used as the sole active ingredient, and can also be used in combination with other drugs for treating diseases related to FXR activity. A combination therapy is achieved by administering the individual therapeutic components simultaneously, separately or sequentially.

### DETAILED DESCRIPTION OF THE INVENTION

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl*, sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "alkenyl" refers to an alkyl group as defined above consisting of at least two carbon atoms and at least one carbon-carbon double bond, such as ethenyl, 1-propenyl, 2-propenyl, 1-, 2- or 3-butenyl and the like. The alkenyl group can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkynyl" refers to an alkyl group as defined above consisting of at least two carbon atoms and at least one carbon-carbon triple bond, such as ethynyl, propynyl, butynyl and the like. The alkynyl group can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one commom carbon atom (called a spiro atom), wherein one or more rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

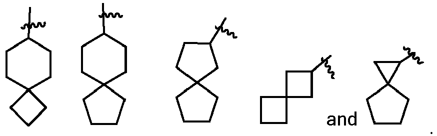

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

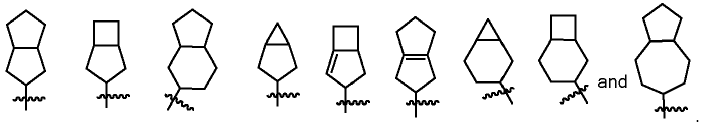

The term "bridged cycloalkyl" refers to a 5 to 20 membered all-carbon polycyclic group, wherein any two rings in the system share two disconnected carbon atoms, one or more rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

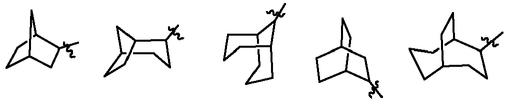

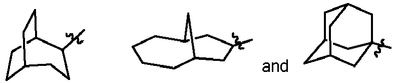

The cycloalkyl ring can be fused to the ring of aryl, heteroaryl or heterocyclyl, wherein the ring linking to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms wherein 1 to 3 atoms are heteroatoms; and most preferably 5 to 7 ring atoms wherein 1 to 2 or 1 to 3 atoms are heteroatoms. Non-limiting examples of monocyclic heterocyclyl include pyrrolidinyl, imidazolyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably, 1,2,5-oxadiazolyl, pyranyl or morpholinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one common atom (called a spiro atom), wherein one or more ring atoms are heteroatoms selected from the group consisting of N, O and S(O)ₘ (wherein m is an integer of 0 to 2), with the remaining ring atoms being carbon atoms, and the rings can contain one or more double bonds, but none of the rings has a completely conjugated π-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

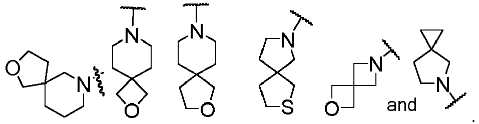

The term "fused heterocyclyl" refers to a 5 to 20 membered polycyclic heterocyclyl group, wherein each ring in the system shares an adjacent pair of atoms with another ring, one or more rings can contain one or more double bonds, but none of the rings has a completely conjugated π-electron system, and one or more ring atoms are heteroatoms selected from the group consisting of N, O and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and preferably a bicyclic or tricyclic fused heterocyclyl, and more preferably a 5-membered/5-membered or 5-membered/6-membered bicyclic fused heterocyclyl. Non-limiting examples of fused heterocyclyl include:

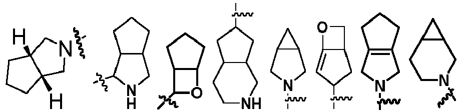

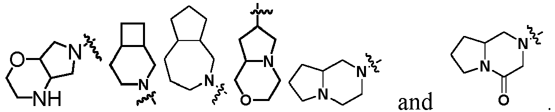

The term "bridged heterocyclyl" refers to a 5 to 14 membered polycyclic heterocyclyl group, wherein any two rings in the system share two disconnected atoms, wherein one or more rings can have one or more double bond(s), but none of the rings has a completely conjugated π-electron system, and one or more ring atoms are heteroatoms selected from the group consisting of N, O and S(O)ₘ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

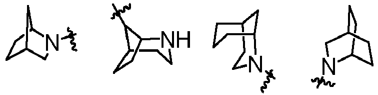

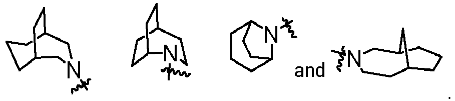

The heterocyclyl ring can be fused to the ring of aryl, heteroaryl or cycloalkyl, wherein the ring bound to the parent structure is heterocyclyl. Non-limiting examples thereof include: 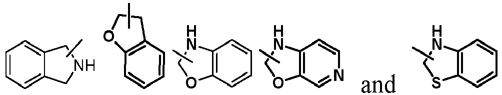 and the like.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include:

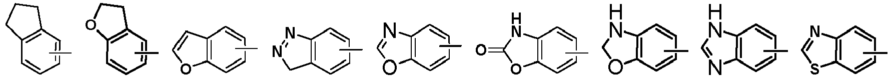

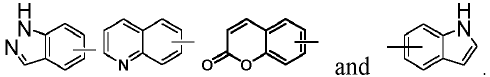

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "heteroaryl" refers to a 5 to 14 membered heteroaromatic system having 1 to 4 heteroatoms selected from the group consisting of O, S and N. The heteroaryl is preferably a 5 to 10 membered heteroaryl having 1 to 3 heteroatom(s), and more preferably a 5 or 6 membered heteroaryl having 1 to 2 heteroatom(s), for example imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, thiazolyl, pyrazolyl, pyrimidinyl or thiazolyl, and more preferably pyrazolyl or thiazolyl. The heteroaryl ring can be fused to the ring of aryl, heterocyclyl or cycloalkyl, wherein the ring bound to the parent structure is heteroaryl ring. Non-limiting examples thereof include:

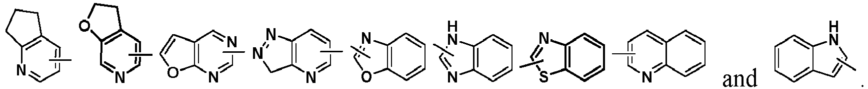

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "haloalkyl" refers to an alkyl group substituted by one or more halogen(s), wherein the alkyl is as defined above.

The term "haloalkoxy" refers to an alkoxy group substituted by one or more halogen(s), wherein the alkoxy is as defined above.

The term "hydroxyalkyl" refers to an alkyl group substituted by hydroxy(s), wherein the alkyl is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to a =O group.

The term "carboxyl" refers to a -C(O)OH group.

The term "thiol" refers to a -SH group.

The term "ester group" refers to a -C(O)O(alkyl) or a -C(O)O(cycloalkyl) group, wherein the alkyl and cycloalkyl are as defined above.

The term "acyl" refers to a compound comprising a -C(O)R group, where R is an alkyl, cycloalkyl, heterocyclyl, aryl or heteroaryl.

The term "sulfonic group" refers to a -S(O)₂OH group.

The term "sulfonic ester" refers to a -S(O)₂O(alkyl) or -S(O)₂O(cycloalkyl) group, wherein the alkyl and cycloalkyl are as defined above.

The term "sulfonyl" refers to a compound comprising a -S(O)₂R group, where R is an alkyl, cycloalkyl, heterocyclyl, aryl or heteroaryl.

The term "aminocarbonyl" refers to a -C(O)-NRR' group, wherein R and R' are each independently hydrogen, alkyl, cycloalkyl, heterocyclyl, aryl or heteroaryl.

The term "aminosulfonyl" or "sulfonylamino" refers to a -S(O)₂-NRR' group, wherein R and R' are each independently hydrogen, alkyl, cycloalkyl, heterocyclyl, aryl or heteroaryl.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the situation of the heterocyclyl being not substituted by an alkyl.

"Substituted" refers to one or more hydrogen atoms in a group, preferably up to 5, and more preferably 1 to 3 hydrogen atoms in a group, are independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

### Synthesis Method of the Compound of the Present Invention

In order to achieve the purpose of the present invention, the present invention applies the following technical solution.

The compound of formula (I) or the salt thereof according to the present invention can be prepared by the following schemes, and the specific preparation methods are as follows.
(1) When Ar is 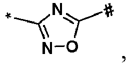 wherein ^{∗} represents the site connected to Cy, and # represents the site connected to the N of the bridged ring; the compound of formula (I) is obtained from compound IA as the starting material according to the method of Scheme 1. 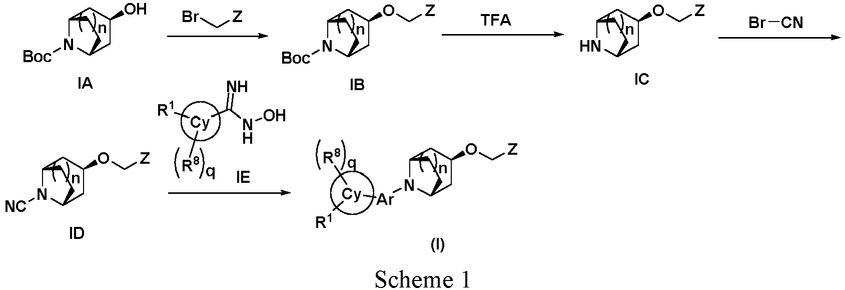 Synthesis process of Scheme 1:
   compound IA is reacted with Z-CH₂-Br under an alkaline condition to obtain compound IB, wherein the alkaline reagent is preferably potassium *tert*-butoxide; compound IB is subjected to a deprotection reaction under an acidic condition to obtain compound IC, wherein the acidic reagent is preferably trifluoroacetic acid; compound IC is reacted with cyanogen bromide under an alkaline condition to obtain compound ID, wherein the alkaline reagent is preferably potassium carbonate; and compound ID and compound IE are subjected to a cyclization reaction in the presence of a condensing agent to obtain the compound of formula (II), wherein the condensing agent is preferably zinc chloride;
   wherein, Z, n, Cy, R¹, R⁸ and q are as defined in formula (I).
(2) When Ar is 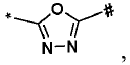 wherein ^{∗} represents the site connected to Cy, and # represents the site connected to the N of the bridged ring; the compound of formula (I) is obtained from compound IF as the starting material according to the method of Scheme 2. 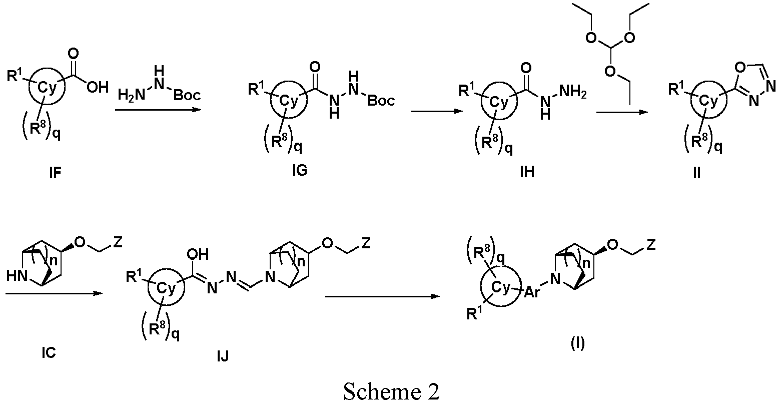 Synthesis process of Scheme 2:
   compound IF and *tert*-butyl carbazate are subjected to a condensation reaction under an alkaline condition in the presence of a condensing agent to obtain compound IG, wherein the alkaline reagent is preferably DMAP, and the condensing agent is preferably EDC; compound IG is subjected to a deprotection reaction under an acidic condition to obtain compound IH, wherein the acidic reagent is preferably trifluoroacetic acid; compound IH and triethyl orthoformate are subjected to a cyclization reaction to obtain compound II; compound II and compound IC are subjected to a condensation reaction under an alkaline condition to obtain compound IJ, wherein the alkaline condition is preferably triethylamine; and compound IJ is subjected to a cyclization reaction in the presence of an oxidizing agent to obtain the compound of formula (I), wherein the oxidizing agent is preferably iodobenzene diacetate;
   wherein, Z, n, Cy, R¹, R⁸ and q are as defined in formula (I).

### EXAMPLES

The present invention will be further described with reference to the following examples, but the examples should not be considered as limiting the scope of the present invention.

The structures of the compounds are identified by nuclear magnetic resonance (NMR) and/or mass spectrometry (MS). NMR shifts (δ) are given in 10⁻⁶ (ppm). NMR is determined by a Brukerdps300 machine. The solvents for determination are deuterated-dimethyl sulfoxide (DMSO-*d₆*), deuterated-chloroform (CDCl₃) and deuterated-methanol (CD₃OD), and the internal standard is tetramethylsilane (TMS).

MS is determined by an 1100 Series LC/MSD Trap (ESI) mass spectrometer (manufacturer: Agilent).

Preparative liquid chromatography is conducted on a lc3000 high performance liquid chromatograph and a lc6000 high performance liquid chromatograph (manufacturer: Beijing Chuangxintongheng science and Technology Co., Ltd.). The chromatographic column used is Daisogel C18 10 µm 60A (20 mm×250 mm).

HPLC is determined by a Shimadzu LC-20AD high pressure liquid chromatograph (Agilent TC-C18 250x4.6 mm 5 µm column) and a Shimadzu LC-2010AHT high pressure liquid chromatograph (Phenomenex C18 250x4.6 mm 5 µm column).

Qingdao Haiyang Chemical GF254 silica gel plate is used for the thin-layer silica gel chromatography (TLC). The dimension of the silica gel plate used in TLC is 0.15 mm to 0.2 mm, and the dimension of the silica gel plate used in product purification is 0.4 mm to 0.5 mm.

Qingdao Haiyang Chemical 100 to 200 mesh and 200 to 300 mesh silica gel is generally used as a carrier for column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Innochem Science & Technology, Nanjing PharmaBlock, Energy Chemical and thye like.

Unless otherwise stated, the reactions are carried out under an argon atmosphere or nitrogen atmosphere.

Argon atmosphere or nitrogen atmosphere means that a reaction flask is equipped with an argon or nitrogen balloon (about 1 L).

Microwave reaction is conducted by a CEM Discover SP type microwave reactor.

Unless otherwise stated, the solution refers to an aqueous solution.

Unless otherwise stated, the reaction temperature is room temperature from 20°C to 30°C.

The progress of the reaction in the examples is monitored by thin layer chromatography (TLC). The developing system includes: A: dichloromethane and methanol system, B: *n*-hexane and ethyl acetate system, C: petroleum ether and ethyl acetate system, D: acetone. The volume ratio of the solvent is adjusted depending on the polarity of the compound.

The eluent system of column chromatography and the developing system of TLC for the purification of the compound include: A: dichloromethane and methanol system, B: petroleum ether, ethyl acetate and dichloromethane system, C: petroleum ether and ethyl acetate system. The volume ratio of the solvent is adjusted depending on the polarity of the compound, and a small amount of an alkaline or acidic reagent such as triethylamine or acetic acid may be added for adjustment.

### Example 1: Preparation of 4-(5-((1R,3r,5S)-3-((5-cyclopropyl-3-(2,6-dichlorophenyl)-isoxazol-4-yl)methoxy)-8-az abicyclo[3.2.1]octan-8-yl)-1,2,4-oxadiazol-3-yl)benzoic acid (1)

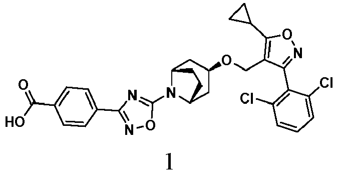

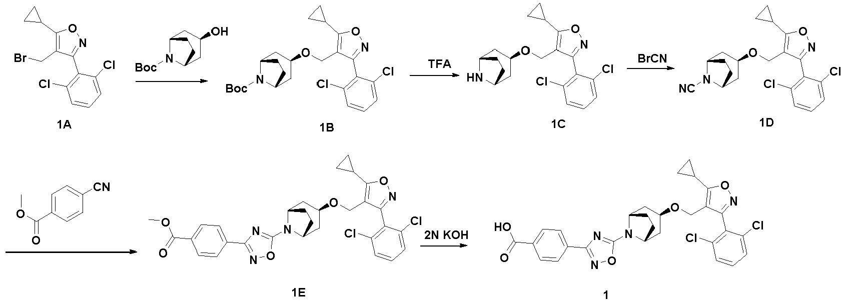

### Step 1: Preparation of tert-butyl (1R,3r,5S)-3-((5-cyclopropyl-3-(2,6-dichlorophenyl)isoxazol-4-yl)methoxy)-8-azabicyc lo[3.2.1]octane-8-carboxylate (1B)

18-Crown-6 (4.95 g, 18.7 mmol), *tert-butyl* (1R,3r,5S)-3-hydroxy-8-azabicyclo[3.2.1]octane-8-carboxylate (4.24 g, 18.7 mmol) and THF (90 mL) were added to a reaction flask, and cooled to 0°C. Potassium *tert*-butoxide (2.86 g, 25.5 mmol) was added to the above mixture and stirred at 0°C for 5 minutes. 4-(Bromomethyl)-5-cyclopropyl-3-(2,6-dichlorophenyl)isoxazole (prepared according to the method disclosed in the patent application WO2011020615) (5.90 g, 17 mmol) was dissolved in 10 mL of THF, and the resulting solution was slowly added dropwise to the above mixture. After completion of the addition, the reaction solution was stirred at room temperature for 2 hours. After completion of the reaction, ethyl acetate (100 mL) and water (100 mL) were added to the reaction solution. The organic phase was washed with saturated brine and water once, dried over anhydrous sodium sulfate and filtered. The filtrate was concentrated under reduced pressure to obtain 9.20 g of the crude title product as a yellow oil, which was used directly in the next step.

### Step 2: Preparation of 4-((((1R,3r,5S)-8-azabicyclo[3.2.1]octan-3-yl)oxy)methyl)-5-cyclopropyl-3-(2,6-dichlor ophenyl)isoxazole (1C)

### Tert-butyl

(1R,3r,5S)-3-((5-cyclopropyl-3-(2,6-dichlorophenyl)isoxazol-4-yl)methoxy)-8-azabicyc lo[3.2.1]octane-8-carboxylate (9.20 g, 18.7 mmol), dichloromethane (36 mL) and trifluoroacetic acid (18 mL) were added to a reaction flask, and stirred at room temperature for 1 hour. After completion of the reaction, the reaction solution was washed with aqueous sodium bicarbonate solution and water once, dried over anhydrous sodium sulfate and filtered. The filtrate was concentrated under reduced pressure, and the resulting residue was purified by silica gel column chromatography (eluent: dichloromethane - dichloromethane: methanol = 10:1) to obtain 4.00 g of the title product as a yellow solid, yield: 54.6%.

### Step 3: Preparation of (1R,3r,5S)-3-((5-cyclopropyl-3-(2,6-dichlorophenyl)isoxazol-4-yl)methoxy)-8-azabicyc 1o[3.2.1]octane-8-carbonitrile (1D)

4-((((1R,3r,5S)-8-Azabicyclo[3.2.1]octan-3-yl)oxy)methyl)-5-cyclopropyl-3-(2,6-d ichlorophenyl)isoxazole (4.00 g, 10.24 mmol), water (80 mL) and potassium carbonate (4.30 g, 30.72 mmol) were added to a reaction flask. Cyanogen bromide (1.20 g, 11.27 mmol) was added to 80 mL of dichloromethane under stirring at room temperature, and the resulting solution was slowly added dropwise to the reaction flask. After completion of the addition, the reaction solution was stirred at room temperature for 2 hours. After completion of the reaction, the reaction solution was washed with IN aqueous hydrochloric acid solution (90 mL) and water (90 mL) once, dried over anhydrous sodium sulfate and filtered. The filtrate was concentrated under reduced pressure to obtain 4.00 g of the title product as a yellow oil, yield: 94.0%.

### Step 4: Preparation of methyl 4-(5-((1R,3r,5S)-3-((5-cyclopropyl-3-(2,6-dichlorophenyl)isoxazol-4-yl)methoxy)-8-aza bicyclo[3.2.1]octan-8-yl)-1,2,4-oxadiazol-3-yl)benzoate (1E)

Methyl p-cyanobenzoate (2.00 g, 12.4 mmol), methanol (40 mL), hydroxylamine hydrochloride (0.87 g, 12.4 mmol) and sodium bicarbonate (1.13 g, 13.4 mmol) were added to a reaction flask. The reaction solution was stirred at room temperature for 0.5 hour, and at 75°C for 3 hours. After completion of the reaction, the reaction solution was poured into 50 mL of ice water, stirred for 10 minutes and filtered. The filter cake was washed with water, and dried to obtain 1.50 g of methyl 4-(*N*-hydroxyformamidino)benzoate as a white solid, yield: 62.2%.

Methyl 4-(*N*-hydroxyformamidino)benzoate (1.00 g, 2.4 mmol), ethyl acetate (30 mL), 0.5N zinc chloride in tetrahydrofuran (18 mL) and (1R,3r,5S)-3-((5-cyclopropyl-3-(2,6-dichlorophenyl)isoxazol-4-yl)methoxy)-8-azabicyc 1o[3.2.1]octane-8-carbonitrile (1.00 g, 5.15 mmol) were added to a reaction flask. The reaction solution was stirred at 50°C for 3 hours, and at 70°C for 18 hours. After completion of the reaction, water (100 mL) and ethyl acetate (100 mL) were added to the reaction solution. The organic phase was washed with water and saturated brine once, dried over anhydrous sodium sulfate and filtered. The filtrate was concentrated under reduced pressure, and the resulting residue was purified by silica gel column chromatography (eluent: *n*-heptane: ethyl acetate=5:1) to obtain 0.60 g of the title product as a white solid, yield: 42.1%.

### Step 5: Preparation of 4-(5-((1R,3r,5S)-3-((5-cyclopropyl-3-(2,6-dichlorophenyl)isoxazol-4-yl)methoxy)-8-aza bicyclo[3.2.1]octan-8-yl)-1,2,4-oxadiazol-3-yl)benzoic acid (1)

### Methyl

4-(5-((1R,3r,5S)-3-((5-cyclopropyl-3-(2,6-dichlorophenyl)isoxazol-4-yl)methoxy)-8-aza bicyclo[3.2.1]octan-8-yl)-1,2,4-oxadiazol-3-yl)benzoate (0.60 g, 1 mmol), methanol (30 mL) and 2N aqueous potassium hydroxide solution (10 mL) were added to a reaction flask, and stirred at 40°C for 18 hours. After completion of the reaction, 100 mL of water was added, and the solution was adjusted to pH=2~3 with hydrochloric acid to precipitate a yellow solid. The mixture was filtered and dried to obtain 580 mg of yellow solid, which was purified by preparative liquid chromatography (eluent: 0%-100% acetonitrile: water solution) to obtain 278 mg of the title product as a light yellow solid, yield: 47.5%.

MS: m/z=581.4 [M+H]⁺.

¹H NMR (300 MHz, DMSO): *δ* ppm 1.14 (m, 2 H), 1.27 (m,2 H), 1.81 (m, 2 H), 2.00 (m, 6 H), 2.33 (m, 1 H), 3.55 (s, 1 H), 4.28 (s, 2 H), 4.37 (m, 2 H), 7.36 (m, 1 H), 7.43 (m, 2 H), 8.11 (m, 2 H), 8.17 (m, 2 H).

### Example 2: Preparation of 2-chloro-4-(5-((1R,3r,5S)-3-((5-cyclopropyl-3-(2,6-dichlorophenyl)isoxazol-4-yl)metho xy)-8-azabicyclo[3.2.1]octan-8-yl)-1,2,4-oxadiazol-3-yl)benzoic acid (2)

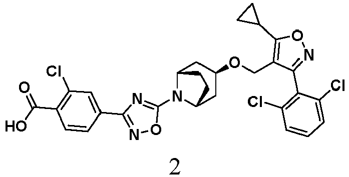

The title compound 2 was obtained in accordance with the same preparation method of Example 1 except for replacing methyl p-cyanobenzoate with methyl 2-chloro-4-cyanobenzoate.

MS: m/z=615.2 [M+H]⁺.

¹H NMR (300 MHz, DMSO): *δ* ppm 1.11 (m, 2 H), 1.28 (m,2 H), 1.68 (m, 2 H), 1.75 (m, 4 H), 1.94 (m, 2 H), 2.35 (m, 1 H), 3.50(m, 1 H), 4.06 (m, 2 H), 4.38 (s, 2 H), 7.63 (m, 3 H), 7.92 (m, 1 H), 8.06 (m, 2 H).

### Example 3: Preparation of 6-(5-((1R,3r,5S)-(3-((5-cyclopropyl-3-(2,6-dichlorophenyl)isoxazol-4-yl)methoxy)-8-az abicyclo[3.2.1]octan-8-yl)-1,2,4-oxadiazol-3-yl)pyridine-2-carboxylic acid (3)

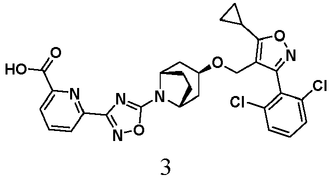

The title compound 3 was obtained in accordance with the same preparation method of Example 1 except for replacing methyl p-cyanobenzoate with methyl 6-cyanopyridine-2-carboxylate.

MS: m/z=582.2 [M+H]⁺.

¹H NMR (300 MHz, DMSO): *δ* ppm 1.11 (m, 4 H), 1.64 (m, 2 H), 1.80 (m, 4 H), 1.94 (m, 2 H), 2.36 (m, 1 H), 3.52(m, 1 H), 4.29 (m, 2 H), 4.37 (s, 2 H), 7.80 (m, 3 H), 8.27 (m, 3 H).

### Example 4: Preparation of 5-(5-((1R,3r,5S)-(3-((5-cyclopropyl-3-(2,6-dichlorophenyl)isoxazol-4-yl)methoxy)-8-az abicyclo[3.2.1]octan-8-yl)-1,2,4-oxadiazol-3-yl)thiophene-2-carboxylic acid (4)

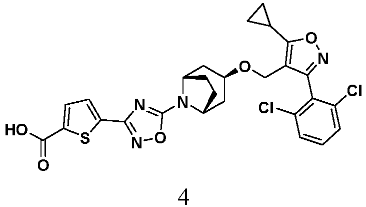

The title compound 4 was obtained in accordance with the same preparation method of Example 1 except for replacing methyl p-cyanobenzoate with methyl 5-cyanothiophene-2-carboxylate.

MS: m/z=587.1 [M+H]⁺.

¹H NMR (300 MHz, DMSO): *δ* ppm 1.16 (m, 2 H), 1.28 (m,2 H), 1.66 (m, 2 H), 1.74 (m, 4 H), 1.85 (m, 2 H), 2.34 (m, 1 H), 3.54(m, 1 H), 4.22 (m, 2 H), 4.28 (s, 2 H), 7.64 (m, 3 H), 7.79 (m, 1 H), 7.90 (m, 1 H).

### Example 5: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,2,4-oxadiazol-3-yl)-3-methylbenzoic acid (5)

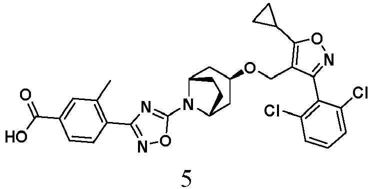

The title compound 5 was obtained in accordance with the same preparation method of Example 1 except for replacing methyl p-cyanobenzoate with methyl 4-cyano-3-methylbenzoate.

MS: m/z=595.4 [M+H]⁺.

¹H NMR (300 MHz, DMSO): *δ* ppm 1.12 (m, 2 H), 1.28 (m,2 H), 1.78 (m, 2 H), 1.86 (m, 4 H), 1.91 (m, 2 H), 2.31 (m, 1 H), 2.65(s, 3 H), 3.58(m, 1 H), 4.06 (m, 2 H), 4.26 (s, 2 H), 7.58 (m, 1 H), 7.66 (m, 2 H), 7.93 (m, 2 H), 8.05 (m, 1 H).

### Example 6: Preparation of 3-(5-((1R,3r,5S)-(3-((5-cyclopropyl-3-(2,6-dichlorophenyl)isoxazol-4-yl)methoxy)-8-az abicyclo[3.2.1]octan-8-yl)-1,2,4-oxadiazol-3-yl)benzoic acid (6)

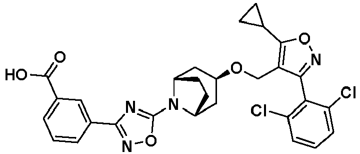

The title compound 6 was obtained in accordance with the same preparation method of Example 1 except for replacing methyl p-cyanobenzoate with methyl *m*-cyanobenzoate.

MS: m/z=581.1 [M+H]⁺.

¹H NMR (300 MHz, DMSO): *δ* ppm 1.11 (m, 4 H), 1.59 (m, 2 H), 1.68 (m, 4 H), 1.98 (m, 2 H), 2.31 (m, 1 H), 2.65(s, 3 H), 3.48(m, 1 H), 4.07 (m, 2 H), 4.28 (s, 2 H), 7.59 (m, 3 H), 7.75 (m, 1 H), 8.25 (m, 2 H), 8.57 (m, 1 H).

### Example 7: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,2,4-oxadiazol-3-yl)-2-methylbenzoic acid (7)

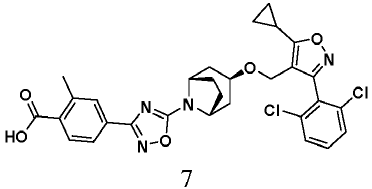

The title compound 7 was obtained in accordance with the same preparation method of Example 1 except for replacing methyl p-cyanobenzoate with methyl 4-cyano-2-methylbenzoate.

MS: m/z=595.4 [M+H]⁺.

¹H NMR (300 MHz, DMSO): *δ* ppm 1.12 (m, 2 H), 1.26 (m,2 H), 1.78 (m, 2 H), 1.76 (m, 4 H), 1.98 (m, 2 H), 2.34 (m, 1 H), 2.59(s, 3 H), 3.48(m, 1 H), 4.08 (m, 2 H), 4.27 (s, 2 H), 7.64 (m, 3 H), 7.95 (m, 3 H).

### Example 8: Preparation of 4-(5-((1R,3r,5S)-3-((5-cyclopropyl-3-(2,6-dichlorophenyl)isoxazol-4-yl)methoxy)-8-aza bicyclo[3.2.1]octan-8-yl)-1,3,4-oxadiazol-2-yl)benzoic acid (8)

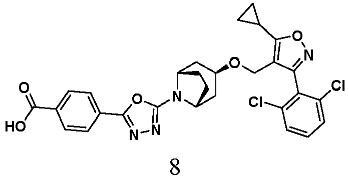

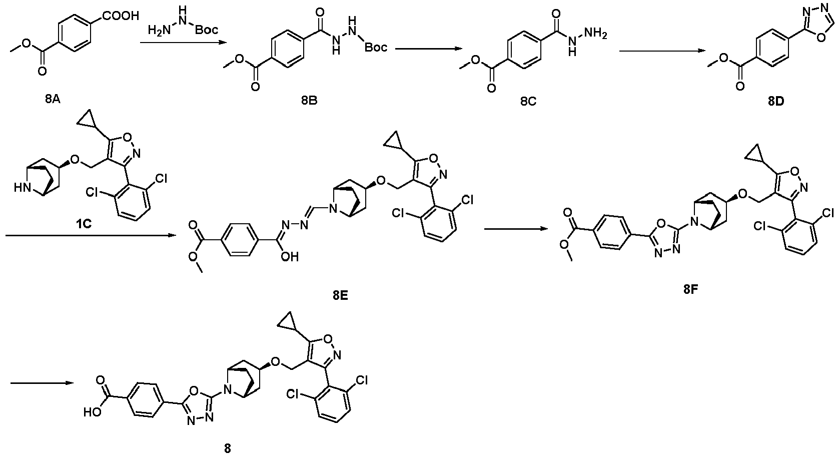

### Step 1: Preparation of tert-butyl 2-(4-(methoxycarbonyl)benzoyl)hydrazine-1-carboxylate (8B)

Monomethyl terephthalate (5.00 g, 27.8 mmol), dichloromethane (80 mL), *tert*-butyl carbazate (4.50 g, 34.4 mmol), DMAP (13.5 g, 111 mmol) and EDCI (7.50 g, 38.9 mmol) were added to a reaction flask, and stirred at room temperature for 20 hours. After completion of the reaction, the reaction solution was extracted with 100 mL of water and 100 mL of dichloromethane. The organic phase was washed with water and saturated brine once, dried over anhydrous sodium sulfate and filtered. The filtrate was concentrated under reduced pressure to obtain 16.30 g of the title product as a light yellow solid, which was used directly in the next step, yield: 100%.

### Step 2: Preparation of methyl 4-(hydrazinecarbonyl)benzoate (8C)

Tert-butyl 2-(4-(methoxycarbonyl)benzoyl)hydrazine-1-carboxylate (18.94 g, 64.4 mmol) and ethyl acetate (50 mL) were added to a reaction flask, followed by the addition of a saturated solution (40 mL) of hydrochloric acid in ethyl acetate at 0°C. The reaction solution was stirred at room temperature for 18 hours. After completion of the reaction, the reaction solution was adjusted to pH=8~9 with saturated sodium bicarbonate solution, filtered and dried to obtain 4.80 g of the title product as a white solid, yield: 38.4%.

### Step 3: Preparation of methyl 4-(1,3,4-oxadiazol-2-yl)benzoate (8D)

Methyl 4-(hydrazinecarbonyl)benzoate (3.00 g, 15.5 mmol) and triethyl orthoformate (11.40 g, 77.3 mmol) were added to a reaction flask, and stirred at 105°C for 16 hours. After completion of the reaction, the reaction solution was concentrated under reduced pressure, and the resulting residue was purified by silica gel column chromatography (eluent: *n*-heptane: ethyl acetate=3:1) to obtain 1.00 g of the title product as a white solid, yield: 31.7%.

### Step 4: Preparation of (Z)-N-((E)-((1R,3r,5S)-3-((5-cyclopropyl-3-(2,6-dichlorophenyl)isoxazol-4-yl)methoxy )-8-azabicyclo[3.2.1]octan-8-yl)methylene)-4-(methoxycarbonyl)benzohydrazonic acid (8E)

Methyl 4-(1,3,4-oxadiazol-2-yl)benzoate (0.50 g, 2.45 mmol), DMF (10 mL), 4-((((1R,3r,5S)-8-azabicyclo[3.2.1]octan-3-yl)oxy)methyl)-5-cyclopropyl-3-(2,6-dichlor ophenyl)isoxazole (1C) (4.50 g, 34.4 mmol) and TEA (2 mL) were added to a reaction flask, and stirred at 90°C for 18 hours. After completion of the reaction, water (40 mL) and dichloromethane (40 mL) were added to the reaction solution. The organic phase was washed with water and saturated brine once, dried over anhydrous sodium sulfate and filtered. The filtrate was concentrated under reduced pressure, and the resulting residue was purified by silica gel column chromatography (eluent: dichloromethane: methanol=20:1) to obtain 0.40 g of the title product as a brown solid, yield: 27.4%.

### Step 5: Preparation of methyl 4-(5-((1R,3r,5S)-3-((5-cyclopropyl-3-(2,6-dichlorophenyl)isoxazol-4-yl)methoxy)-8-aza bicyclo[3.2.1]octan-8-yl)-1,3,4-oxadiazol-2-yl)benzoate (8F)

(Z)-N-((E)-((1R,3r,5S)-3-((5-Cyclopropyl-3-(2,6-dichlorophenyl)isoxazol-4-yl)met hoxy)-8-azabicyclo[3.2.1]octan-8-yl)methylene)-4-(methoxycarbonyl)benzohydrazonic acid (8E) (0.30 g, 0.5 mmol), dichloromethane (10 mL) and iodobenzene diacetate (0.24 g, 0.756 mmol) were added to a reaction flask, and stirred at room temperature for 1 hour. After completion of the reaction, the reaction solution was concentrated under reduced pressure, and the resulting residue was purified by preparative liquid chromatography (eluent: 0%-100% acetonitrile: water) to obtain 0.135 g of the title product as a white solid, yield: 45.1%.

### Step 6: Preparation of 4-(5-((1R,3r,5S)-3-((5-cyclopropyl-3-(2,6-dichlorophenyl)isoxazol-4-yl)methoxy)-8-aza bicyclo[3.2.1]octan-8-yl)-1,3,4-oxadiazol-2-yl)benzoic acid (8)

### Methyl

4-(5-((1R,3r,5S)-3-((5-cyclopropyl-3-(2,6-dichlorophenyl)isoxazol-4-yl)methoxy)-8-aza bicyclo[3.2.1]octan-8-yl)-1,3,4-oxadiazol-2-yl)benzoate (8F) (0.135 g, 0.23 mmol), methanol (10 mL), water (5 mL), THF (15 mL) and lithium hydroxide (33 mg, 1.36 mmol) were added to a reaction flask, and stirred at 40°C for 24 hours. After completion of the reaction, the reaction solution was adjusted to pH=1~2 with hydrochloric acid, followed by the addition of water (40 mL) and ethyl acetate (40 mL). The organic phase was washed with water and saturated brine once, dried over anhydrous sodium sulfate and filtered. The filtrate was concentrated under reduced pressure, and the resulting residue was purified by preparative liquid chromatography (eluent: 0%-100% acetonitrile: water) to obtain 56 mg of the title product as a white solid, yield: 42.5%.

MS: m/z=581 [M+H]⁺.

¹H NMR (300 MHz, DMSO): *δ* ppm 1.11 (m, 4 H), 1.64 (m, 2 H), 1.76 (m, 4 H), 1.91 (m, 2 H), 2.38 (m, 1 H), 3.46(m, 1 H), 4.04 (m, 2 H), 4.28 (s, 2 H), 7.62 (m, 3 H), 8.18 (m, 4 H).

### Example 9: Preparation of 2-chloro-4-(5-((1R,3r,5S)-3-((5-cyclopropyl-3-(2,6-dichlorophenyl)isoxazol-4-yl)metho xy)-8-azabicyclo[3.2.1]octan-8-yl)-1,3,4-oxadiazol-2-yl)benzoic acid (9)

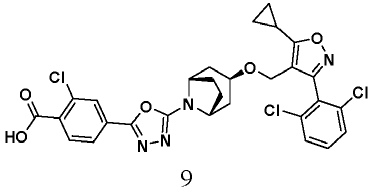

The title compound 9 was obtained in accordance with the same preparation method of Example 8 except for replacing monomethyl terephthalate with 3-chloro-4-(methoxycarbonyl)benzoic acid.

MS: m/z=615.2 [M+H]⁺.

¹H NMR (300 MHz, DMSO): *δ* ppm 1.11 (m, 4 H), 1.64 (m, 2 H), 1.76 (m, 4 H), 1.91 (m, 2 H), 2.36 (m, 1 H), 3.48(m, 1 H), 4.07 (m, 2 H), 4.27 (s, 2 H), 7.59 (m, 1 H), 7.67 (m, 2 H), 7.92 (m, 1 H), 8.04 (m, 1 H), 8.08 (m, 1 H).

### Example 10: Preparation of 6-(5-((1R,3r,5S)-3-((5-cyclopropyl-3-(2,6-dichlorophenyl)isoxazol-4-yl)methoxy)-8-aza bicyclo[3.2.1]octan-8-yl)-1,3,4-oxadiazol-2-yl)pyridine-2-carboxylic acid (10)

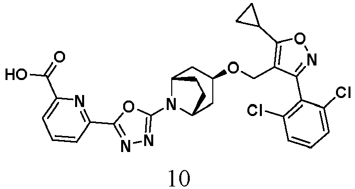

The title compound 10 was obtained in accordance with the same preparation method of Example 8 except for replacing monomethyl terephthalate with monomethyl 2,6-pyridinedicarboxylate.

MS: m/z=582.2 [M+H]⁺.

¹H NMR (300 MHz, DMSO): *δ* ppm 1.11 (m, 4 H), 1.64 (m, 2 H), 1.75 (m, 4 H), 1.90 (m, 2 H), 2.35 (m, 1 H), 3.48(m, 1 H), 4.09 (m, 2 H), 4.27 (s, 2 H), 7.72 (m, 3 H), 8.25 (m, 3 H).

### Example 11: Preparation of 5-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)thiophene-2-carboxylic acid (11)

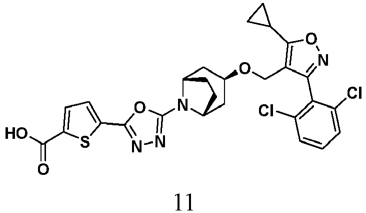

The title compound 11 was obtained in accordance with the same preparation method of Example 8 except for replacing monomethyl terephthalate with methyl 5-carboxylthiophene-2-carboxylate.

MS: m/z=587.1 [M+H]⁺.

¹H NMR (300 MHz, DMSO): *δ* ppm 1.11 (m, 4 H), 1.60 (m, 2 H), 1.74 (m, 4 H), 1.85 (m, 2 H), 2.35 (m, 1 H), 3.47(m, 1 H), 4.02 (m, 2 H), 4.26 (s, 2 H), 7.61 (m, 3 H), 7.79 (m, 1 H), 7.90 (m, 1 H).

### Example 12: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)-2-fluorobenzoic acid (12)

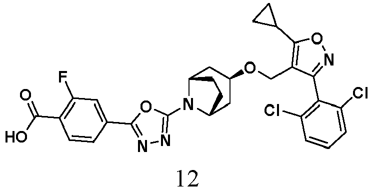

The title compound 12 was obtained in accordance with the same preparation method of Example 8 except for replacing monomethyl terephthalate with 3-fluoro-4-(methoxycarbonyl)benzoic acid.

MS: m/z=599.4 [M+H]⁺.

¹H NMR (300 MHz, DMSO): *δ* ppm 1.11 (m, 4 H), 1.64 (m, 2 H), 1.75 (m, 4 H), 1.91 (m, 2 H), 2.32 (m, 1 H), 3.49 (m, 1 H), 4.06 (m, 2 H), 4.26 (s, 2 H), 7.63 (m, 3 H), 7.92 (m, 2 H), 8.06 (m, 1 H).

### Example 13: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)-2-methoxybenzoic acid (13)

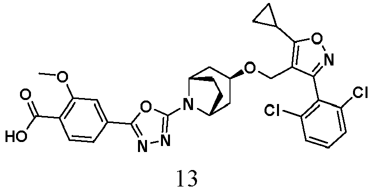

The title compound 13 was obtained in accordance with the same preparation method of Example 8 except for replacing monomethyl terephthalate with 3-methoxy-4-(methoxycarbonyl)benzoic acid.

MS: m/z=611.2 [M+H]⁺.

¹H NMR (300 MHz, DMSO): *δ* ppm 1.11 (m, 4 H), 1.64 (m, 2 H), 1.76 (m, 4 H), 1.91 (m, 2 H), 2.32 (m, 1 H), 3.48(m, 1 H), 3.92(m, 3 H), 4.07 (m, 2 H), 4.26 (s, 2 H), 7.61 (m, 5 H), 7.77 (m, 1 H).

### Example 14: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)-3-methylbenzoic acid (14)

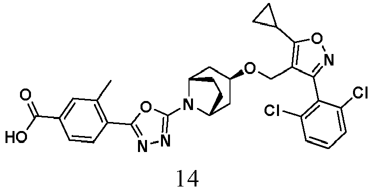

The title compound 14 was obtained in accordance with the same preparation method of Example 8 except for replacing monomethyl terephthalate with 2-methyl-4-(methoxycarbonyl)benzoic acid.

MS: m/z=595.4 [M+H]⁺.

¹H NMR (300 MHz, DMSO): *δ* ppm 1.11 (m, 4 H), 1.59 (m, 2 H), 1.76 (m, 4 H), 1.91 (m, 2 H), 2.37 (m, 1 H), 2.65(s, 3 H), 3.48(m, 1 H), 4.06 (m, 2 H), 4.28 (s, 2 H), 7.56 (m, 1 H), 7.66 (m, 2 H), 7.90 (m, 1 H), 7.96 (m, 1 H), 8.05 (m, 1 H).

### Example 15: Preparation of 3-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)benzoic acid (15)

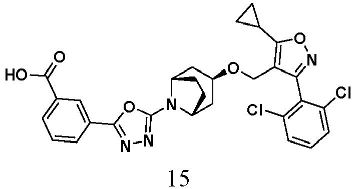

The title compound 15 was obtained in accordance with the same preparation method of Example 8 except for replacing monomethyl terephthalate with 3-(methoxycarbonyl)benzoic acid.

MS: m/z=581.1 [M+H]⁺.

¹H NMR (300 MHz, DMSO): *δ* ppm 1.11 (m, 4 H), 1.60 (m, 2 H), 1.67 (m, 4 H), 1.88 (m, 2 H), 2.32 (m, 1 H), 3.48(m, 1 H), 4.09 (m, 2 H), 4.26 (s, 2 H), 7.59 (m, 3 H), 7.77 (m, 1 H), 8.23 (m, 2 H), 8.51 (m, 1 H).

### Example 16: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)-3-fluorobenzoic acid (16)

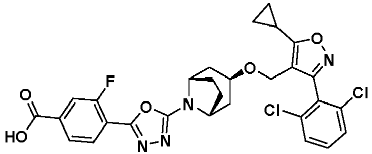

The title compound 16 was obtained in accordance with the same preparation method of Example 8 except for replacing monomethyl terephthalate with 2-fluoro-4-(methoxycarbonyl)benzoic acid.

MS: m/z=599.4 [M+H]⁺.

¹H NMR (300 MHz, DMSO): *δ* ppm 1.11 (m, 4 H), 1.60 (m, 2 H), 1.62 (m, 4 H), 1.75 (m, 2 H), 2.31 (m, 1 H), 3.54(m, 1 H), 4.04 (m, 2 H), 4.27 (s, 2 H), 7.64 (m, 4 H), 7.81 (m, 1 H), 7.92 (m, 1H).

### Example 17: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)-2-methylbenzoic acid (17)

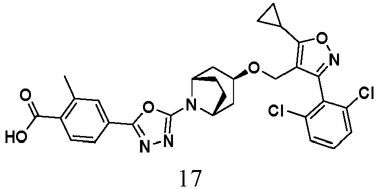

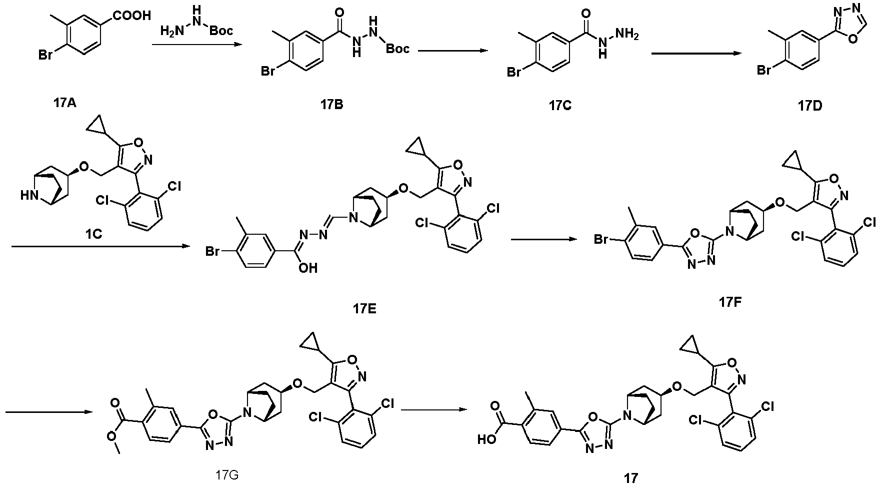

### Step 1: Preparation of tert-butyl 2-(4-bromo-3-methylbenzoyl)hydrazine-1-carboxylate (17B)

4-Bromo-3-methylbenzoic acid (5.98 g, 27.8 mmol), dichloromethane (80 mL), *tert*-butyl carbazate (4.50 g, 34.4 mmol), DMAP (13.5 g, 111 mmol) and EDCI (7.50 g, 38.9 mmol) were added to a reaction flask, and stirred at room temperature for 20 hours. After completion of the reaction, the reaction solution was extracted with 100 mL of water and 100 mL of dichloromethane. The organic phase was washed with water and saturated brine once, dried over anhydrous sodium sulfate and filtered. The filtrate was concentrated under reduced pressure to obtain 8.90 g of the title product as a light yellow solid, which was used directly in the next step, yield: 97.1%.

### Step 2: Preparation of 4-bromo-3-methylbenzohydrazide (17C)

Tert-butyl 2-(4-bromo-3-methylbenzoyl)hydrazine-1-carboxylate (8.90 g, 27.1 mmol) and ethyl acetate (50 mL) were added to a reaction flask, followed by the addition of a saturated solution (40 mL) of hydrochloric acid in ethyl acetate at 0°C. The reaction solution was stirred at room temperature for 18 hours. After completion of the reaction, the reaction solution was adjusted to pH=8~9 with saturated sodium bicarbonate solution, filtered and dried to obtain 5.60 g of the title product as a white solid, yield: 90.5%.

### Step 3: Preparation of 2-(4-bromo-3-methylphenyl)-1,3,4-oxadiazole (17D)

4-Bromo-3-methylbenzohydrazide (5.60 g, 24.5 mmol) and triethyl orthoformate (50 mL) were added to a reaction flask, and stirred at 105°C for 16 hours. After completion of the reaction, the reaction solution was concentrated under reduced pressure, and the resulting residue was purified by silica gel column chromatography (eluent: *n*-heptane: ethyl acetate=3:1) to obtain 4.20 g of the title product as a white solid, yield: 71.8%.

### Step 4: Preparation of (Z)-4-bromo-N-((E)-((1R,3r,5S)-3-((5-cyclopropyl-3-(2,6-dichlorophenyl)isoxazol-4-yl) methoxy)-8-azabicyclo[3.2.1]octan-8-yl)methylene)-3-methylbenzohydrazonic acid (17E)

2-(4-Bromo-3-methylphenyl)-1,3,4-oxadiazole (0.50 g, 2.10 mmol), DMF (10 mL), 4-((((1R,3r,5S)-8-azabicyclo[3.2.1]octan-3-yl)oxy)methyl)-5-cyclopropyl-3-(2,6-dichlor ophenyl)isoxazole (1C) (0.82 g, 2.10 mmol) and DIPEA (5 mL) were added to a reaction flask, and stirred at 110°C for 18 hours. After completion of the reaction, water (40 mL) and dichloromethane (40 mL) were added to the reaction solution. The organic phase was washed with water and saturated brine once, dried over anhydrous sodium sulfate and filtered. The filtrate was concentrated under reduced pressure, and the resulting residue was purified by silica gel column chromatography (eluent: dichloromethane: methanol=20:1) to obtain 0.40 g of the title product as a brown solid, yield: 30.2%.

### Step 5: Preparation of 2-(4-bromo-3-methylphenyl)-5-((1R,3r,5S)-3-((5-cyclopropyl-3-(2,6-dichlorophenyl)iso xazol-4-yl)methoxy)-8-azabicyclo[3.2.1]octan-8-yl)-1,3,4-oxadiazole (17F)

(Z)-4-Bromo-N-((E)-((1R,3r,5S)-3-((5-cyclopropyl-3-(2,6-dichlorophenyl)isoxazol -4-yl)methoxy)-8-azabicyclo[3.2.1]octan-8-yl)methylene)-3-methylbenzohydrazonic acid (0.40 g, 0.63 mmol), dichloromethane (10 mL) and iodobenzene diacetate (0.31 g, 0.95 mmol) were added to a reaction flask, and stirred at room temperature for 1 hour. After completion of the reaction, the reaction solution was concentrated under reduced pressure, and the resulting residue was purified by silica gel column chromatography (eluent: petroleum ether: ethyl acetate=2:1) to obtain 0.35 g of the title product as a yellow oil, yield: 87.8%.

### Step 6: Preparation of methyl 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)-2-methylbenzoate (17G)

2-(4-Bromo-3-methylphenyl)-5-((1R,3r,5S)-3-((5-cyclopropyl-3-(2,6-dichlorophen yl)isoxazol-4-yl)methoxy)-8-azabicyclo[3.2.1]octan-8-yl)-1,3,4-oxadiazole (0.35 g, 0.56 mmol), Pd(dppf)Cl₂ (41 mg, 0.056 mmol), sodium acetate (92 mg, 1.12 mmol), methanol (10 mL) and DMF (3 mL) were added to a high pressure reaction flask (TGYF, Huaou). The reaction solution was purged with carbon monoxide three times to remove air, and stirred at 0.5 mPa and 80°C for 16 hours. After completion of the reaction, the reaction solution was concentrated under reduced pressure, and the resulting residue was purified by silica gel column chromatography (eluent: petroleum ether: ethyl acetate=2:1) to obtain 0.20 g of the title product as a yellow oil, yield: 59.0%.

### Step 7: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)-2-methylbenzoic acid (17)

### Methyl

4-(5-(((1R,3r,5S)-(3-((5-cyclopropyl-3-(2,6-dichlorophenyl)isoxazol-4-yl)methoxy)-8-a zabicyclo[3.2.1]octan-8-yl)-1,3,4-oxadiazol-2-yl)-2-methylbenzoate (0.20 g, 0.33 mmol), methanol (10 mL), water (5 mL), THF (15 mL) and lithium hydroxide (33 mg, 1.36 mmol) were added to a reaction flask, and stirred at 40°C for 24 hours. After completion of the reaction, the reaction solution was adjusted to pH=1~2 with hydrochloric acid, followed by the addition of water (40 mL) and ethyl acetate (40 mL). The organic phase was washed with water and saturated brine once, dried over anhydrous sodium sulfate and filtered. The filtrate was concentrated under reduced pressure, and the resulting residue was purified by preparative liquid chromatography (eluent: 0%-100% acetonitrile: water) to obtain 76 mg of the title product as a white solid, yield: 38.8%.

MS: m/z=595.3 [M+H]⁺.

¹H NMR (300 MHz, DMSO): *δ* ppm 1.14 (m, 4 H), 1.65 (m, 2 H), 1.79 (m, 4 H), 1.97 (m, 2 H), 2.36 (m, 1 H), 2.51 (m, 3 H), 3.50 (m, 1 H), 4.18 (m, 2 H), 4.28 (s, 2 H), 7.62 (m, 5 H), 7.92 (m, 1 H).

### Example 18: Preparation of 5-(5-((1R,3r,5S)-3-((5-cyclopropyl-3-(2,6-dichlorophenyl)isoxazol-4-yl)methoxy)-8-aza bicyclo[3.2.1]octan-8-yl)-1,3,4-oxadiazol-2-yl)pyridine-carboxylic acid (18)

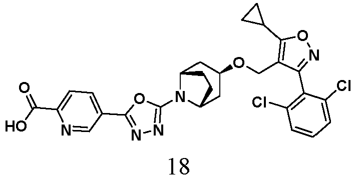

The title compound 18 was obtained in accordance with the same preparation method of Example 8 except for replacing monomethyl terephthalate with methyl pyridine-2,5-dicarboxyl-2-carboxylate.

MS: m/z=582.5 [M+H]⁺.

¹H NMR (300 MHz, DMSO): *δ* ppm 1.14 (m, 4 H), 1.65 (m, 2 H), 1.79 (m, 4 H), 1.95 (m, 2 H), 2.35 (m, 1 H), 3.53 (m, 1 H), 4.19 (m, 2 H), 4.27 (s, 2 H), 7.59 (m, 3 H), 8.15 (m, 1 H), 8.38 (m, 1 H), 9.14 (m, 1 H).

### Example 19: Preparation of 6-(5-((1R,3r,5S)-3-((5-cyclopropyl-3-(2,6-dichlorophenyl)isoxazol-4-yl)methoxy)-8-aza bicyclo[3.2.1]octan-8-yl)-1,3,4-oxadiazol-2-yl)nicotinic acid (19)

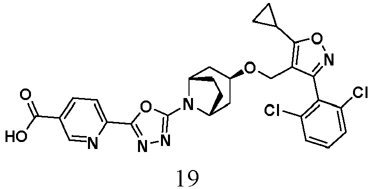

The title compound 19 was obtained in accordance with the same preparation method of Example 8 except for replacing monomethyl terephthalate with 5-(methoxycarbonyl)-2-pyridine-carboxylic acid.

MS: m/z=582.5 [M+H]⁺.

¹H NMR (300 MHz, DMSO): *δ* ppm 1.11 (m, 4 H), 1.66 (m, 2 H), 1.79 (m, 4 H), 1.95 (m, 2 H), 2.35 (m, 1 H), 3.53 (m, 1 H), 4.20 (m, 2 H), 4.27 (s, 2 H), 7.62 (m, 3 H), 8.12 (m, 1 H), 8.39 (m, 1 H), 9.14 (m, 1 H).

### Example 20: Preparation of 5-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)thiophene-3-carboxylic acid (20)

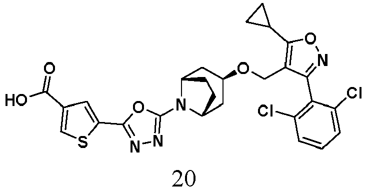

The title compound 20 was obtained in accordance with the same preparation method of Example 17 except for replacing 4-bromo-3-methylbenzoic acid with 4-bromothiophene-2-carboxylic acid.

MS: m/z=587.7 [M+H]⁺.

¹H NMR (300 MHz, DMSO): *δ* ppm 1.11 (m, 4 H), 1.61 (m, 2 H), 1.76 (m, 4 H), 1.85 (m, 2 H), 2.33 (m, 1 H), 3.47 (m, 1 H), 4.12 (m, 2 H), 4.25 (s, 2 H), 7.58 (m, 3 H), 7.77 (m, 1 H), 8.39 (m, 1 H).

### Example 21: Preparation of 4-(5-((1R,3r,5S)-(3-((5-cyclopropyl-3-(2,6-dichlorophenyl)isoxazol-4-yl)methoxy)-8-az abicyclo[3.2.1]octan-8-yl)-1,3,4-oxadiazol-2-yl)thiophene-2-carboxylic acid (21)

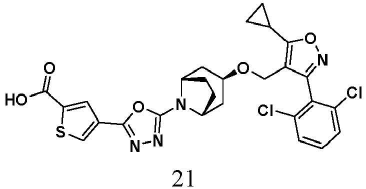

The title compound 21 was obtained in accordance with the same preparation method of Example 17 except for replacing 4-bromo-3-methylbenzoic acid with 5-bromo-3-thiophene-carboxylic acid.

MS: m/z=587.4 [M+H]⁺.

¹H NMR (300 MHz, DMSO): *δ* ppm 1.13 (m, 4 H), 1.68 (m, 2 H), 1.77 (m, 4 H), 1.85 (m, 2 H), 2.33 (m, 1 H), 3.47 (m, 1 H), 4.13 (m, 2 H), 4.26 (s, 2 H), 7.64 (m, 3 H), 7.94 (m, 1 H), 8.34 (m, 1 H).

### Example 22: Preparation of 4-(5-((1R,3r,5S)-3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)benzoic acid (22)

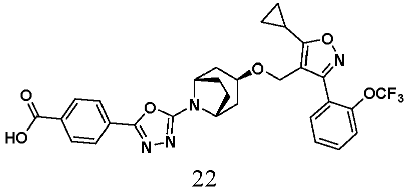

The title compound 22 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519).

MS: m/z=597.7 [M+H]⁺.

¹H NMR (300 MHz, DMSO): *δ* ppm 1.13 (m, 4 H), 1.66 (m, 2 H), 1.80 (m, 4 H), 1.95 (m, 2 H), 2.33 (m, 1 H), 3.53 (m, 1 H), 4.17 (m, 2 H), 4.33 (s, 2 H), 7.82 (m, 4 H), 7.96 (m, 2 H), 8.08 (m, 2 H).

### Example 23: Preparation of 2-chloro-4-(5-((1R,3r,5S)-3-((5-cyclopropyl-3-(2-(trifluoromethoxy)phenyl)isoxazol-4-yl)methoxy)-8-azabicyclo[3.2.1]octan-8-yl)-1,3,4-oxadiazol-2-yl)benzoic acid (23)

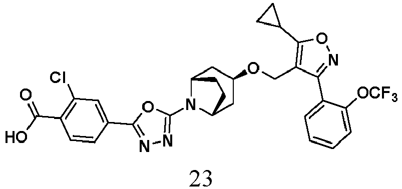

The title compound 23 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with 3-chloro-4-(methoxycarbonyl)benzoic acid.

MS: m/z=631.5[M+H]⁺.

¹H NMR (300 MHz, DMSO): *δ* ppm 1.13 (m, 4 H), 1.66 (m, 2 H), 1.79 (m, 4 H), 1.99 (m, 2 H), 2.33 (m, 1 H), 3.51 (m, 1 H), 4.20 (m, 2 H), 4.33 (s, 2 H), 7.55 (m, 2 H), 7.68 (m, 2 H), 7.89 (m, 2 H), 7.94 (m, 1 H).

### Example 24: Preparation of 6-(5-((1R,3r,5S)-3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)pyridine-2-carboxylic acid (24)

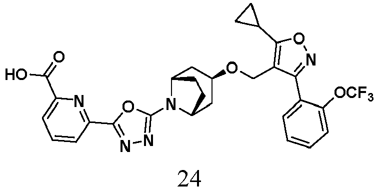

The title compound 24 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with monomethyl 2,6-pyridinedicarboxylate.

MS: m/z=598.5 [M+H]⁺.

¹H NMR (300 MHz, DMSO): *δ* ppm 1.13 (m, 4 H), 1.66 (m, 2 H), 1.79 (m, 4 H), 1.98 (m, 2 H), 2.33 (m, 1 H), 3.51 (m, 1 H), 4.20 (m, 2 H), 4.32 (s, 2 H), 7.62 (m, 4 H), 8.25 (m, 3 H).

### Example 25: Preparation of 5-(5-((1R,3r,5S)-(3-((5-cyclopropyl-3-(2-(trifluoromethoxy)phenyl)isoxazol-4-yl)metho xy)-8-azabicyclo[3.2. 1]octan-8-yl)-1,3,4-oxadiazol-2-yl)thiophene-2-carboxylic acid (25)

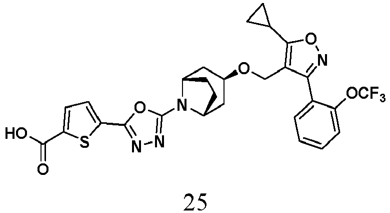

The title compound 25 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with methyl 5-carboxylthiophene-2-carboxylate.

MS: m/z=603.4 [M+H]⁺.

¹H NMR (300 MHz, DMSO): *δ* ppm 1.11 (m, 4 H), 1.65 (m, 2 H), 1.74 (m, 4 H), 1.79 (m, 2 H), 2.33 (m, 1 H), 3.53(m, 1 H), 4.14 (m, 2 H), 4.32 (s, 2 H), 7.65 (m, 6 H).

### Example 26: Preparation of 4-(5-((1R,3r,5S)-(3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)-2-fluorobenzoic acid (26)

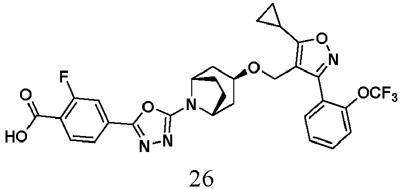

The title compound 26 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with 3-fluoro-4-(methoxycarbonyl)benzoic acid.

MS:m/z=615.3 [M+H]⁺.

¹H NMR (300 MHz, DMSO): *δ* ppm 1.11 (m, 4 H), 1.66 (m, 2 H), 1.79 (m, 4 H), 1.95 (m, 2 H), 2.32 (m, 1 H), 3.52 (m, 1 H), 4.15 (m, 2 H), 4.33 (s, 2 H), 7.62 (m, 2 H), 7.80 (m, 2 H), 7.89 (m, 2 H), 8.02 (m, 1 H).

### Example 27: Preparation of 4-(5-((1R,3r,5S)-(3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)-3-methylbenzoic acid (27)

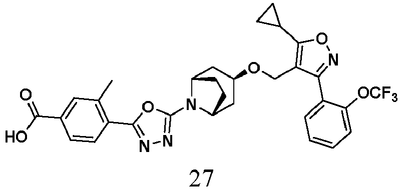

The title compound 27 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with 2-methyl-4-(methoxycarbonyl)benzoic acid.

MS:m/z=611.4 [M+H]⁺.

¹H NMR (300 MHz, DMSO): *δ* ppm 1.16 (m, 4 H), 1.72 (m, 2 H), 1.77 (m, 4 H), 2.00 (m, 2 H), 2.43 (m, 1 H), 2.59 (s, 3 H), 3.54 (m, 1 H), 4.22 (m, 2 H), 4.30 (s, 2 H), 7.63 (m, 2 H), 7.71 (m, 2 H), 7.95 (m, 3 H).

### Example 28: Preparation of 3-(5-((1R,3r,5S)-(3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)benzoic acid (28)

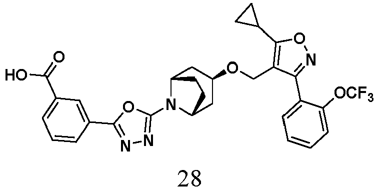

The title compound 28 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with 3-(methoxycarbonyl)benzoic acid.

MS: m/z=597.2 [M+H]⁺.

¹H NMR (300 MHz, DMSO): *δ* ppm 1.11 (m, 4 H), 1.66 (m, 2 H), 1.79 (m, 4 H), 1.94 (m, 2 H), 2.33 (m, 1 H), 3.53 (m, 1 H), 4.17 (m, 2 H), 4.32 (s, 2 H), 7.65 (m, 5 H), 8.05 (m, 2 H), 8.35 (m, 1 H).

### Example 29: Preparation of 4-(5-((1R,3r,5S)-(3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)-3-fluorobenzoic acid (29)

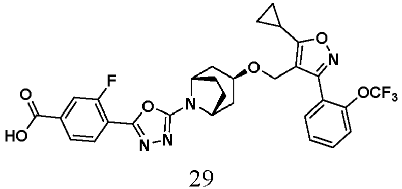

The title compound 29 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with 2-fluoro-4-(methoxycarbonyl)benzoic acid.

MS: m/z=615.5 [M+H]⁺.

¹H NMR (300 MHz, DMSO): *δ* ppm 1.11 (m, 4 H), 1.66 (m, 2 H), 1.79 (m, 4 H), 1.90 (m, 2 H), 2.31 (m, 1 H), 3.52 (m, 1 H), 4.15 (m, 2 H), 4.32 (s, 2 H), 7.57 (m, 2 H), 7.67 (m, 2 H), 7.85 (m, 2 H), 8.02 (m, 1 H).

### Example 30: Preparation of 4-(5-((1R,3r,5S)-(3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)-2-methylbenzoic acid (30)

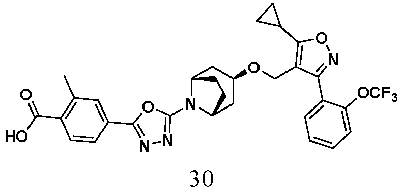

The title compound 30 was obtained in accordance with the same preparation method of Example 17 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519).

MS: m/z=611.4 [M+H]⁺.

¹H NMR (300 MHz, DMSO): *δ* ppm 1.11 (m, 4 H), 1.67 (m, 2 H), 1.80 (m, 4 H), 2.08 (m, 2 H), 2.33 (m, 1 H), 2.54 (m, 1 H), 3.54 (m, 1 H), 4.19 (m, 2 H), 4.34 (s, 2 H), 7.67 (m, 6 H), 7.93 (m, 1 H).

### Example 31: Preparation of 5-(5-((1R,3r,5S)-3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)pyridine-carboxylic acid (31)

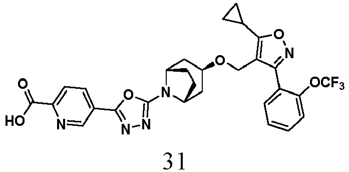

The title compound 31 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with 6-(methoxycarbonyl)nicotinic acid.

MS: m/z=598.5 [M+H]⁺.

¹H NMR (300 MHz, DMSO): *δ* ppm 1.10 (m, 4 H), 1.68 (m, 2 H), 1.80 (m, 4 H), 1.98 (m, 2 H), 2.35 (m, 1 H), 3.53 (m, 1 H), 4.20 (m, 2 H), 4.34 (s, 2 H), 7.59 (m, 4 H), 8.15 (m, 1 H), 8.36 (m, 1 H), 9.14 (m, 1 H).

### Example 32: Preparation of 6-(5-((1R,3r,5S)-3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)nicotinic acid (32)

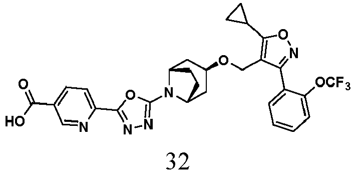

The title compound 32 was obtained in accordance with the same preparation method of Example 8 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing monomethyl terephthalate with 5-(methoxycarbonyl)-2-pyridine-carboxylic acid.

MS: m/z=598.5 [M+H]⁺.

¹H NMR (300 MHz, DMSO): *δ* ppm 1.13 (m, 4 H), 1.69 (m, 2 H), 1.81 (m, 4 H), 1.95 (m, 2 H), 2.35 (m, 1 H), 3.53 (m, 1 H), 4.18 (m, 2 H), 4.28 (s, 2 H), 7.63 (m, 4 H), 8.12 (m, 1 H), 8.39 (m, 1 H), 9.14 (m, 1 H).

### Example 33: Preparation of 5-(5-((1R,3r,5S)-3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)thiophene-3-carboxylic acid (33)

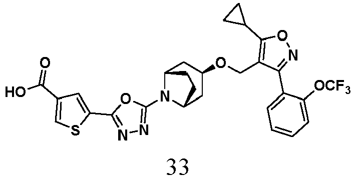

The title compound 33 was obtained in accordance with the same preparation method of Example 17 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing 4-bromo-3-methylbenzoic acid with 4-bromothiophene-2-carboxylic acid.

MS: m/z=603.3 [M+H]⁺.

¹H NMR (300 MHz, DMSO): *δ* ppm 1.13 (m, 4 H), 1.66 (m, 2 H), 1.79 (m, 4 H), 1.85 (m, 2 H), 2.35 (m, 1 H), 3.34 (m, 1 H), 4.15 (m, 2 H), 4.33 (s, 2 H), 7.57 (m, 4 H), 7.79 (m, 1 H), 8.38 (m, 1 H).

### Example 34: Preparation of 4-(5-((1R,3r,5S)-(3-((5-cyclopropyl-3-(2-(trifluoromethoxy)phenyl)isoxazol-4-yl)metho xy)-8-azabicyclo[3.2.1]octan-8-yl)-1,3,4-oxadiazol-2-yl)thiophene-2-carboxylic acid (34)

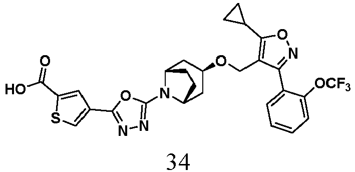

The title compound 34 was obtained in accordance with the same preparation method of Example 17 except for replacing 4-((((1R,3r,5S)-8-azabicyclo[3.2.1]octan-3-yl)oxy)methyl)-5-cyclopropyl-3-(2,6-dichlor ophenyl)isoxazole (1C) with 4-((((1R,3r,5S)-8-azabicyclo[3.2.1]octan-3-yl)oxy)methyl)-5-cyclopropyl-3-(2-(trifluor omethoxy)phenyl)isoxazole (prepared according to the method disclosed in the patent application WO2012087519) and replacing 4-bromo-3-methylbenzoic acid with 5-bromo-3-thiophene-carboxylic acid.

MS: m/z=603.4 [M+H]⁺.

¹H NMR (300 MHz, DMSO): *δ* ppm 1.17 (m, 4 H), 1.72 (m, 2 H), 1.85 (m, 4 H), 1.93 (m, 2 H), 2.56 (m, 1 H), 3.53 (m, 1 H), 4.18 (m, 2 H), 4.38 (s, 2 H), 7.61 (m, 2 H), 7.70 (m, 2 H), 8.01 (m, 1 H), 8.41 (m, 1 H).

### Biological Assay of the Compound of the Present Invention

### Test Example 1: Assay of the FXR agonistic activity of the compound of the present invention

The FXR agonistic activity of the compound of the present invention was evaluated using the luciferase assay.

The experimental process comprises the follows. The luc2P-GAL4-HEK293 stably transfected cell line (the cell line was established by transfecting pGL4.35 plasmid with HEK293 cells which was then subjected to Hygromycin B screening. The cell contained 9xGAL4 UAS and firefly luciferase reporter gene. The ligand-activated LBD-GAL4 DBD fusion protein can enter the nucleus to bind to 9×GAL4 UAS and activate the transcription of the downstream luciferase reporter gene.) was transferred to a 96-well plate with 1×10⁴ cells per well. pBIND-FXR (Which was constructed by inserting FXR-LBD into pFN26A-BIND hRluc-neo Flexi^{®} vector. The plasmid was 7.5 kb in size, Amp resistant, contained FXR-LBD, and can express FXR-LBD and GAL4 DBD fusion proteins.) was transfected using X-tremeGENE HP transfection reagent. The ratio of plasmid to transfection reagent was 1 µg: 2 µl. The specific amout was 100 ng of plasmid and 0.2 µl of X-tremeGENE HP transfection reagent per well. The test compounds (the highest concentration of each compound was 30 µM, 3-fold dilution, a total of 10 concentrations) were added to induce the expression of luciferase. After 24 hours, the 96-well plate containing the test cells was taken out from the incubator, and placed at room temperature. Dual-Glo^{®} Luciferase Reagent with the volume equal to the volume of the medium in the well (80 µl/well) was added to each well and mixed well. The plate was incubated at room temperature for 20 minutes to allow the cells to be fully lysed. All the liquid in the plate was transferred to a 96-well microtiter plate, and the firefly luciferin value was measured with a multi-function plate reader (manufacturer: Bio Tek (USA); model: Synergy4). Dual-Glo^{®} Stop & Glo^{®} Reagent with the volume equal to the volume of the initial medium (80 µl/well) was added to each well and mixed well. The plate was incubated at room temperature for 10 minutes, and the renilla luciferin value was measured. The measurement order of renilla fluorescence should be the same as that of firefly fluorescence. Curve fitting and EC₅₀ calculation were conducted using GraphPad Prism 5 software by nonlinear regression method. The fitting equation is: Y=Bottom + (Top-Bottom)/(1+10^{∧}((LogEC₅₀-X)^{∗}HillSlope)), wherein EC50 refers to the semi-effective concentration, Top refers to the maximum effect, Bottom refers to the blank effect, and HillSlope refers to the slope. The activities of the compounds are shown in Table 1.

In Table 1, A means that the compound has a FXR agonistic activity of ECso < 25 nM; B means that the EC₅₀₌ 25 nM to 50 nM; C means that the EC₅₀₌ 50 nM to 100 nM; D means that the EC₅₀₌ 100 nM to 500 nM; and E means that the EC₅₀ > 500 nM.

**Table 1. FXR agonistic activity of the compounds of the present invention**

| Examples | FXR agonistic activity |
|---|---|
| Example 1 | A |
| Example 2 | A |
| Example 3 | A |
| Example 4 | A |
| Example 5 | A |
| Example 6 | A |
| Example 7 | A |
| Example 8 | A |
| Example 9 | C |
| Example 10 | D |
| Example 11 | D |
| Example 12 | D |
| Example 13 | A |
| Example 14 | A |
| Example 15 | A |
| Example 16 | C |
| Example 17 | A |
| Example 18 | D |
| Example 19 | E |
| Example 20 | C |
| Example 21 | D |
| Example 22 | A |
| Example 23 | D |
| Example 24 | E |
| Example 25 | D |
| Example 26 | D |
| Example 27 | A |
| Example 28 | A |
| Example 29 | D |
| Example 30 | A |
| Example 31 | D |
| Example 32 | E |
| Example 33 | D |
| Example 34 | E |

Conclusion: as shown in Table 1 above, the compounds of the present invention show FXR agonistic activity *in vitro.*

### Test Example 2: Efficacy of the compound of the present invention on the cholestasis rat model induced by 1-naphthyl isothiocyanate (ANIT)

Animals: SD rats, male, 7 to 8 weeks old, weight: 220 to 240 g, purchased from Beijing Vital River Laboratory Animal Technology Co., Ltd., SPF grade, Certificate No.: SCXK (Beijing) 2016-0011, license issuing administration: Beijing Municipal Commission of Science and Technology.

Sample formulation: The compound of the present invention was added to DMSO, and dissolved completely by ultrasound. 0.5% CMC-Na was added to constant volume (DMSO: 0.5% CMC-Na=1:99), and the solution was homogenized by ultrasound for later use.

After 3 to 5 days of acclimation, the animals were divided into 20 groups according to body weight: normal group, model group, Example 8 (3 mg/kg/d, 10 mg/kg/d) group, Example 11 (3 mg/kg/d, 10 mg/kg/d) group, Example 12 (3 mg/kg/d, 10 mg/kg/d) group, Example 14 (3 mg/kg/d, 10 mg/kg/d) group, Example 20 (3 mg/kg/d, 10 mg/kg/d) group, Example 22 (3 mg/kg/d, 10 mg/kg/d) group, Example 26 (3 mg/kg/d, 10 mg/kg/d) group, Example 28 (3 mg/kg/d, 10 mg/kg/d) group, and Example 29 (3 mg/kg/d, 10 mg/kg/d) group, with 10 rats for each group. Intragastric administration was conducted with an administration volume of 10 mL/kg, once a day, for a total of 4 days. On Day 2, ANIT (1-naphthyl isothiocyanate, Macklin, purity: 98%, item number: N814658, CAS number: 551-06-4, specification: 5 g/bottle, storage conditions: 2 to 8 degrees, batch number: C10116101) with a dose of 50 mg/kg and an administration volume of 5 mL/kg was administered intragastrically once to each animal (except for animals in the normal group) 4 hours after the administration. The animals were fasted on Day 4 morning. The animals were anesthetized by isoflurane inhalation using a small animal anesthesia machine. Blood was collected from the venous plexus behind the eyeball using a capillary glass tube (about 1 ml per animal), and then left to stand for about one hour. It was centrifuged at 3500 rpm for 10 minutes to obtain the serum. ALT, AST, GGT and TBA indexes as well as the plasma concentration of the compound were determined using an AU480 automatic biochemical analysis system (BECKMAN COULTER). Liver was collected, weighed, and cutted into pieces. Normal saline was added (liver weight: normal saline = 1:2), and the mixture was homogenized and freezed. The concentration of the compound in liver was determined using an ultra performance liquid chromatograph (Shimadzu, LC 30AD) and triple quadrupole mass spectrometer (AB, TQ5500).

The efficacy of the compound of the present invention on the cholestasis rat induced by ANIT is shown in Table 2 below. The plasma concentration and liver concentration of the compound of the present invention after 4 days of administration in the cholestasis rat induced by ANIT are shown in Table 3, Table 4 and Table 5 below.

**Table 2. Efficacy of the compound of the present invention on the cholestasis rat induced by ANIT**

| | ALT | AST | GGT | TBA |
|---|---|---|---|---|
| Normal | 43.4±7.3^{∗∗} | 127.0±11.5^{∗∗} | 1.3±0.5^{∗∗} | 7.9±2.6^{∗∗} |
| Model | 344.7±131.5 | 964.0±274.7 | 5.4±1.7 | 234.3±82.1 |
| Example 8 (3mg/kg/d) | 164.8±85.0^{∗∗} | 401.8±172.8^{∗∗} | 2.5±0.7^{∗∗} | 100.0±99.8 |
| Example 8 (10mg/kg/d) | 79.9±98.7^{∗∗} | 243.5±192.6^{∗∗} | 2.0±0.5^{∗∗} | 32.9±68.0^{∗∗} |
| Example 11 (3mg/kg/d) | 276.3±118.0 | 761.3±253.7 | 4.9±0.9 | 243.4±72.4 |
| Example 11 (10mg/kg/d) | 180.3±107.8^{∗} | 466.1±242.4^{∗} | 3.7±1.5^{∗} | 190.3±140.7 |
| Example 12 (3mg/kg/d) | 122.5±89.7^{∗∗} | 314.2±237.9^{∗} | 2.2±0.8^{∗∗} | 69.0±66.7^{∗} |
| Example 12 (10mg/kg/d) | 54.7±27.3^{∗∗} | 139.3±34.6^{∗∗} | 1.9±0.5^{∗∗} | 25.4±14.3^{∗∗} |
| Example 14 (3mg/kg/d) | 220.3±104.0^{∗} | 643.3±239.3^{∗} | 4.6±1.3 | 235.8±80.1 |
| Example 14 (10mg/kg/d) | 45.5±11.9^{∗∗} | 181.4±20.8^{∗∗} | 1.7±0.4^{∗∗} | 20.3±12.3^{∗∗} |
| Example 20 (3mg/kg/d) | 141.5±101.4^{∗} | 400.2±304.4^{∗} | 2.8±1.2^{∗} | 119.5±124.7 |
| Example 20 (10mg/kg/d) | 50.9±22.4^{∗∗} | 139.8±58.4^{∗∗} | 2.6±0.5^{∗∗} | 16.1±8.0^{∗∗} |
| Example 22 (3mg/kg/d) | 100.8±90.9^{∗∗} | 324.8±313.1^{∗∗} | 2.7±1.7^{∗} | 73.3±103.2^{∗} |
| Example 22 (10mg/kg/d) | 44.5±8.1^{∗∗} | 163.3±21.3^{∗∗} | 1.8±0.5^{∗∗} | 27.4±13.6^{∗∗} |
| Example 26 (3mg/kg/d) | 62.2±27.6^{∗∗} | 187.7±50.4^{∗∗} | 1.9±0.8^{∗∗} | 23.2±10.1^{∗∗} |
| Example 26 (10mg/kg/d) | 50.0±11.2^{∗∗} | 162.3±27.7^{∗∗} | 1.4±0.7^{∗∗} | 35.3±13.2^{∗∗} |
| Example 28 (3mg/kg/d) | 147.1±66.2 | 342.7±153.6^{∗} | 2.3±0.5^{∗∗} | 66.2±58.4^{∗} |
| Example 28 (10mg/kg/d) | 46.6±12.1^{∗∗} | 150.8±28.7^{∗∗} | 1.9±0.5^{∗∗} | 20.5±6.0^{∗∗} |
| Example 29 (3mg/kg/d) | 82.0±44.5^{∗} | 256.0±168.8^{∗} | 2.3±1.1^{∗} | 51.7±65.2^{∗} |
| Example 29 (10mg/kg/d) | 77.8±69.5^{∗∗} | 189.4±76.9^{∗∗} | 1.9±0.4^{∗∗} | 26.8±13.6^{∗∗} |

| | | | | |
|---|---|---|---|---|
| Note: ^{∗} means comparison with the model group, P<0.05; and ^{∗∗} means comparison with the model group, P<0.01. | | | | |

**Table 3. Results of the plasma concentration of the compound of the present invention after 4 days of administration**

| Groups | Plasma concentration of the compound (ng/mL) | |
|---|---|---|
| | 4h | 2h |
| Example 8 (3mg/kg/d) | 129.7±119.5 | 44.6±36.9 |
| Example 8 (10mg/kg/d) | 16.7±16.4 | 50.4±74.4 |
| Example 11 (3mg/kg/d) | 340.0±253.2 | 400.9±98.4 |
| Example 11 (10mg/kg/d) | 664.3±738.8 | 1161.2±1037.2 |
| Example 14 (3mg/kg/d) | 172.1±75.3 | 168.1±111.2 |
| Example 14 (10mg/kg/d) | 250.0±72.4 | 402.4±237.5 |
| Example 22 (3mg/kg/d) | 278.0±192.3 | 440.5±480.6 |
| Example 22 (10mg/kg/d) | 567.2±242.0 | 902.7±348.4 |

**Table 4. Results of the liver concentration of the compound of the present invention after 4 days of administration**

| Groups | Liver concentration of the compound (ng/g) | |
|---|---|---|
| | 4h | 2h |
| Example 8 (3mg/kg/d) | 1189.5±162.3 | 751.9±419.0 |
| Example 8 (10mg/kg/d) | 1190.2±264.9 | 986.5±355.3 |
| Example 11 (3mg/kg/d) | 1,519.8±979.6 | 1,587.4±835.6 |
| Example 11 (10mg/kg/d) | 3,614.0±1,961.3 | 5,660.8±1,816.7 |
| Example 14 (3mg/kg/d) | 1,148.1±329.7 | 1,235.4±747.5 |
| Example 14 (10mg/kg/d) | 5,093.6±2,197.5 | 7,844.0±3,854.2 |
| Example 22 (3mg/kg/d) | 3,089.5±570.2 | 3,762.1±1,640.3 |
| Example 22 (10mg/kg/d) | 9,659.1±965.8 | 12,349.5±3,988.9 |

**Table 5. Results of the liver concentration/ the plasma concentration of the compound of the present invention after 4 days of administration**

| Groups | Liver concentration/ plasma concentration of the compound | |
|---|---|---|
| | 4h | 2h |
| Example 8 (3mg/kg/d) | 61.0±107.0 | 43.9±65.2 |
| Example 8 (10mg/kg/d) | 105.1±48.6 | 54.9±37.0 |
| Example 11 (3mg/kg/d) | 6.2±3.9 | 3.9±1.4 |
| Example 11 (10mg/kg/d) | 15.5±12.7 | 9.5±9.1 |
| Example 14 (3mg/kg/d) | 7.1±1.6 | 7.4±1.5 |
| Example 14 (10mg/kg/d) | 19.8±7.0 | 21.0±4.4 |
| Example 22 (3mg/kg/d) | 14.4±6.5 | 16.1±10.9 |
| Example 22 (10mg/kg/d) | 19.2±6.9 | 14.2±2.1 |

Conclusion: The compounds of Examples 8, 11, 12, 14, 20, 22, 26, 28, 29 of the present invention significantly reduce the level of ALT, AST, GGT and TBA in the serum of ANIT rat, indicating that the compounds of the present invention can significantly improve cholestasis in ANIT rat. Moreover, these compounds have high liver targeting.

### Test Example 3: Assay of the effect of the compound of the present invention on hERG channel

**Test reagents:**

| Reagents | Supplier | Item No. | Batch No. |
|---|---|---|---|
| DMEM | Gibco | 11995-065 | 1897371 |
| Fetal bovine serum | Gibco | 10091148 | 1872295 |
| G418 | Amresco | E859 | 17J065302 |
| TrypLE^{™} Express | Gibco | 12604021 | 1810976 |
| Dimethyl sulfoxide | Sigma | D4540 | BCBW5664 |
| Cisapride | Sigma | C4740 | 0000021445 |
| Potassium aspartate | Sigma | A6558 | WXBC4890V |
| Sodium chloride | Sigma | S5886 | SLBS7447 |
| Potassium chloride | Sigma | P5405 | SLBR2609V |
| Ethylene glycol-bis(2-aminoethyl ether) | Sigma | E3889 | SLBR7504V |
| 4-Hydroxyethylpiperazine ethanesulfonic acid | Santa Cruz | SC-29097A | J3015/C2817 |
| Magnesium chloride | Sigma | M2393 | SLBP9770V |
| Glucose | Sigma | G8270 | SLBR5156V |
| Calcium chloride | Sigma | C7902 | SLBV3136 |
| Sodium dihydrogen phosphate | GENERAL-REAGENT | G21298B | P1297763 |
| Adenosine sodium triphosphate | Sigma | A2383 | SLBT7818 |
| Phosphate buffered saline (PBS) | Takara | T900 | 2301 |
| hERG stable expression cell line | Creacell | A-0320 | N/A |

**Test Instruments:**

| Name | Supplier | Model |
|---|---|---|
| Amplifier | HEKA (Germany) | EPC10 |
| Micromanipulator | Sutter Instruments (USA) | MP285 |
| Electrode puller | Sutter Instruments (USA) | P97 |
| Microscope | Olympus (Japan) | IX71 |
| Capillary glass tube | Sutter Instruments (USA) | BF150-86-10 |
| Data collection and analysis software | HEKA (Germany) | Patchmaster & IGOR |

Intracellular fluid and extracellular fluid:
Extracellular fluid: 140 mM NaCl, 3.5 mM KCl, 1 mM MgCl₂, 2 mM CaCl₂, 10 mM glucose, 10 mM HEPES, 1.25 mM NaH₂PO₄, pH=7.4 (adjusted by NaOH).
Intracellular fluid: 20 mM KCl, 115 mM K-aspartic acid, 1 mM MgCl₂, 5 mM EGTA, 10 mM HEPES, 2 mM Na₂-ATP, pH=7.2 (adjusted byKOH).

Formulation of the test sample:
The stock solution of the compound of Example 8 of the present invention (30.26 mM solution in DMSO) was successively diluted with 10 mL of extracellular fluid to obtain 0.3 µM, 1 µM, 3 µM, 0 µM, and 30 µM solutions. The solubility of the test sample was visually inspected, and the test sample was completely dissolved without visible precipitation.

Formulation of Cisapride (positive control):
10 mg of Cisapride was dissolved in 2066.29 µL of dimethyl sulfoxide (DMSO) to obtain a 10 mM stock solution. The Cisapride stock solution was successively diluted with dimethyl sulfoxide (DMSO) to obtain a total of 4 concentrations of 1 µM, 10 µM, 100 µM and 1 mM. Before the test, 10 µL of the diluent at each concentration was added to 10 mL of extracellular fluid to ensure that the DMSO concentration was 0.1%. The final working concentration of Cisapride was 1 nM, 10 nM, 100 nM and 1 µM. The solubility of Cisapride was visually inspected, and Cisapride was completely dissolved without visible precipitation.

Cell culture:
The HEK293 cell line stably expressing the hERG potassium channel (supplier Creacell, catalog number A-0302) was cultured in DMEM medium containing 10% fetal bovine serum and 0.8 mg/mL G418. The culture temperature was 37°C, and the carbon dioxide concentration was 5%.

Cell passage: The old medium was removed, and the cells were washed with PBS once. 1 mL of TrypLE^{™} expression solution was added, and the cells were incubated at 37°C for 0.5 minutes. When the cells detached from the bottom of the dish, 5 mL of 37°C pre-warmed complete medium (DMEM medium containing 10% fetal bovine serum and 0.8 mg/mL G418) was added. The cell suspension was gently pipetted to separate the aggregated cells. The cell suspension was transferred to a sterile centrifuge tube, and centrifuged at 1000 rpm for 5 minutes to collect the cells. Expansion or maintenance culture, the cells were seeded in a 6 cm cell culture dish, and each cell culture dish was seeded with 2.5×10⁵ cells (final volume: 5 mL). In order to maintain the electrophysiological activity of the cell, the cell density must not exceed 80%. The cells were separated by TrypLE^{™} expression solution, and 3×10³ cells were placed on a cover slide and cultured in a 24-well plate (final volume: 500 µL). Membrane clamp test was carried out after 18 hours.

Experiment process of electrophysiological record:
The voltage stimulus protocol of the whole-cell membrane clamp for recording the whole-cell hERG potassium current is as follows. When a whole-cell sealing was formed, the membrane clamp was disposed at -80 mV. The clamp voltage was depolarized from -80 mV to +30 mV for 2.5 seconds, and then quickly maintained at -50 mV for 4 seconds, which can excite the tail current of the hERG channel. -50 mV was used for leakage current detection in the test. The data was collected repeatly every 10 seconds to observe the effect of the compound on hERG tail current. The test data was collected by EPC-10 amplifier (HEKA) and stored in PatchMaster (HEKA) software.

A capillary glass tube (BF150-86-10, Sutter Instruments) was prepared into a recording electrode using a microelectrode puller (P97, Sutter Instruments). Microelectrode manipulator (MP285, Sutter Instruments) was manipulated under an inverted microscope (IX71, Olympus) to contact the recording electrode to the cell, and suction was applied under negative pressure to form a GΩ seal. After forming the GΩ seal, a rapid capacitance compensation was performed, then a negative pressure was applied to break the cell membrane and create a whole-cell recording mode. A slow capacitor compensation was performed, and the membrane capacitance and series resistance were recorded without leakage compensation.

When the hERG current of the whole-cell record was stable, administration was conducted. Each concentration was tested for 5 minutes (or until the current was stable) before testing the next concentration. Multiple concentrations were tested for each test compound. The cover slide seeded with the cell was placed in a recording bath of an inverted microscope. The test compound and the compound-free extracellular fluid flowed through the recording chamber by gravity perfusion from low concentrations to high concentrations to act on the cell. Liquid exchange was performed using a vacuum pump in the record. For each cell, the current detected in the compound-free extracellular fluid was used as the control. Three cells were repeatly tested independently. All electrophysiological experiments were carried out at room temperature.

Data quality standard:
The following standards were used to determine whether the data can be accepted or not:
(1) Series resistance ≤ 20 MΩ
(2) Sealing resistance ≥ 1 GΩ
(3) Start tail current peak ≥ 400 pA
(4) The start tail current peak was higher than the activation current peak
(5) There was no significant spontaneous attenuation of the tail current (spontaneous attenuation within 5 minutes was less than 5%)
(6) There was no significant leakage current when the membrane potential was -80 mV (leakage current ≤ 100 pA)

The results of the inhibitory effect of the compound of Example 8 of the present invention on hERG channel and the semi-inhibiting concentration (IC₅₀) are shown in Table 6 below.

**Table 6. Inhibitory effect of the compound of Example 8 of the present invention on hERG channel and the semi-inhibiting concentration (IC₅₀)**

| Compound | hERG current inhibition ratio (%) | | | | | n | IC₅₀ |
|---|---|---|---|---|---|---|---|
| | 0.3 µM | 1 µM | 3 uM | 10 µM | 30 µM | | |
| Example 8 | -2.37±0.62 | -3.09±0.30 | 0.48±1.78 | 12.65±2.25 | 31.67±1.86 | 3 | >30µM |

The experimental results show that the compound of Example 8 of the present invention has no inhibitory effect on hERG channel.

## Claims

1. A compound of formula (I) or a mesomer, racemate, enantiomer, diastereomer thereof, or mixture thereof, or a prodrug thereof, or a pharmaceutically acceptable salt thereof, 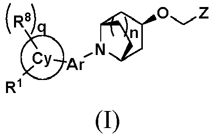 wherein:
Z is selected from the group consisting of 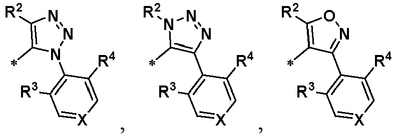 and 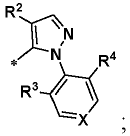
wherein X is CH, CF, N or NO;
R² is selected from the group consisting of hydrogen, halogen, alkyl and cycloalkyl, wherein the alkyl and the cycloalkyl are each optionally further substituted by one or more substituent(s) selected from the group consisting of halogen, hydroxy, alkyl and alkoxy;
R³ and R⁴ are each independently selected from the group consisting of hydrogen, halogen, alkyl and alkoxy, wherein the alkyl and the alkoxy are each optionally further substituted by one or more halogen;
Ar is a 5-membered or 6-membered aryl or a heteroaryl;
Cy is an aryl or a heteroaryl;
R¹ is selected from the group consisting of -(CH₂)ₘ-R⁵ and -O(CH₂)ₘ-R⁵, wherein the -(CH₂)ₘ- and -O(CH₂)ₘ- are each optionally further substituted by one or more substituent(s) selected from the group consisting of halogen, cyano, hydroxy, oxo, alkyl, haloalkyl, alkoxy, haloalkoxy, CO₂H and SO₃H;
R⁵ is selected from the group consisting of hydrogen, halogen, cyano, nitro, alkyl, cycloalkyl, heterocyclyl, aryl, heteroaryl, OR⁶, NR⁶R⁷, -CO₂R⁶, -C(O)R⁶, -C(O)NR⁶R⁷, -N(R⁶)C(O)R⁷, -C(O)NR⁶SO₂R⁷, -S(O)ₚR⁶, -S(O)ₚNR⁶R⁷, -N(R⁶)S(O)ₚR⁷ and -S(O)ₚNR⁶COR⁷, wherein the alkyl, cycloalkyl, heterocyclyl, aryl and heteroaryl are each optionally further substituted by one or more substituent(s) selected from the group consisting of halogen, amino, nitro, cyano, hydroxy, thiol, carboxyl, ester group, oxo, alkyl, haloalkyl, alkoxy, haloalkoxy, alkenyl, alkynyl, cycloalkyl, halocycloalkyl, heterocyclyl, aryl and heteroaryl;
R⁶ and R⁷ are each independently selected from the group consisting of hydrogen, halogen, hydroxy, alkyl, cycloalkyl, heterocyclyl, aryl and heteroaryl, wherein the alkyl, cycloalkyl, heterocyclyl, aryl and heteroaryl are each optionally further substituted by one or more substituent(s) selected from the group consisting of halogen, amino, nitro, cyano, hydroxy, thiol, carboxyl, ester group, oxo, alkyl, alkoxy, cycloalkyl, heterocyclyl, aryl and heteroaryl;
or, R⁶ and R⁷, together with the nitrogen atom to which they are attached, form a nitrogen-containing heterocyclyl, wherein the nitrogen-containing heterocyclyl is optionally further substituted by one or more substituent(s) selected from the group consisting of halogen, amino, nitro, cyano, oxo, hydroxy, thiol, carboxyl, ester group, alkyl, alkoxy, cycloalkyl, heterocyclyl, aryl and heteroaryl;
each R⁸ can be identical or different and are each independently selected from the group consisting of halogen, amino, nitro, cyano, hydroxy, thiol, carboxyl, ester group, alkyl, haloalkyl, alkoxy, haloalkoxy, alkenyl, alkynyl, cycloalkyl, halocycloalkyl, heterocyclyl, aryl and heteroaryl;
n is 0, 1 or 2;
m is an integer from 0 to 6;
p is 0, 1 or 2;
q is an integer from 0 to 4;
with the proviso that Ar is not 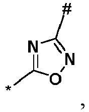 wherein ^{∗} represents the site connected to Cy, and # represents the site connected to the N of the bridged ring.

2. The compound of formula (I) or the mesomer, racemate, enantiomer, diastereomer thereof, or mixture thereof, or the prodrug thereof, or the pharmaceutically acceptable salt thereof according to claim 1, being a compound of formula (II) or a mesomer, racemate, enantiomer, diastereomer thereof, or mixture thereof, or a prodrug thereof, or a pharmaceutically acceptable salt thereof, 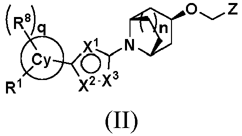 wherein,
X¹, X² and X³ are each independently selected from the group consisting of C, N, O and S, and preferably N and O;
Z, n, Cy, R¹, R⁸ and q are as defined in claim 1.

3. The compound of formula (I) or the mesomer, racemate, enantiomer, diastereomer thereof, or mixture thereof, or the prodrug thereof, or the pharmaceutically acceptable salt thereof according to claim 1 or 2,
wherein,
Z is 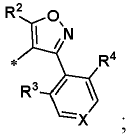
X is selected from the group consisting of CH and N;
R² is selected from the group consisting of hydrogen, halogen, alkyl and cycloalkyl, preferably C₁-C₆ alkyl and C₃-C₆ cycloalkyl, and more preferably cyclopropyl, wherein the alkyl and cycloalkyl are each optionally further substituted by one or more substituent(s) selected from the group consisting of halogen, hydroxy, alkyl and alkoxy;
R³ and R⁴ are each independently selected from the group consisting of hydrogen, halogen, alkyl, haloalkyl, alkoxy and haloalkoxy, and preferably hydrogen, halogen, C₁-C₆ haloalkyl and C₁-C₆ haloalkoxy.

4. The compound of formula (I) or the mesomer, racemate, enantiomer, diastereomer thereof, or mixture thereof, or the prodrug thereof, or the pharmaceutically acceptable salt thereof according to any one of claims 1 to 3,
wherein, n is 1.

5. The compound of formula (I) or the mesomer, racemate, enantiomer, diastereomer thereof, or mixture thereof, or the prodrug thereof, or the pharmaceutically acceptable salt thereof according to any one of claims 1 to 4,
wherein,
Cy is a C₅-C₆ aryl or a 5- to 6-membered heteroaryl, and preferably phenyl, pyridyl, pyrimidinyl, pyrazinyl, thiazolyl, furyl, imidazolyl or pyrazolyl.

6. The compound of formula (I) or the mesomer, racemate, enantiomer, diastereomer thereof, or mixture thereof, or the prodrug thereof, or the pharmaceutically acceptable salt thereof according to any one of claims 1 to 5,
wherein,
R¹ is selected from the group consisting of -(CH₂)ₘ-R⁵ and -O(CH₂)ₘ-R⁵;
R⁵ is selected from the group consisting of hydrogen, halogen, alkyl, OR⁶, NR⁶R⁷, -CO₂R⁶, -C(O)R⁶, -C(O)NR⁶R⁷, -N(R⁶)C(O)R⁷, -C(O)NR⁶SO₂R⁷, -S(O)ₚR⁶, -S(O)ₚ NR⁶R⁷, -N(R⁶)S(O)ₚR⁷ and -S(O)ₚNR⁶COR⁷, preferably -C(O)R⁶, -C(O)NR⁶R⁷, -S(O)ₚR⁶ and -S(O)ₚNR⁶R⁷, more preferably -C(O)R⁶ and -S(O)ₚNR⁶R⁷, and even more preferably -COOH;
R⁶ and R⁷ are each independently selected from the group consisting of hydrogen, halogen, hydroxy, alkyl, cycloalkyl, heterocyclyl, aryl and heteroaryl, wherein the alkyl, cycloalkyl, heterocyclyl, aryl and heteroaryl are each optionally further substituted by one or more substituent(s) selected from the group consisting of halogen, amino, nitro, cyano, hydroxy, thiol, carboxyl, ester group, oxo, alkyl, alkoxy, cycloalkyl, heterocyclyl, aryl and heteroaryl;
or, R⁶ and R⁷, together with the nitrogen atom to which they are attached, form a nitrogen-containing heterocyclyl, wherein the nitrogen-containing heterocyclyl is optionally further substituted by one or more substituent(s) selected from the group consisting of halogen, amino, nitro, cyano, oxo, hydroxy, thiol and carboxyl;
m is an integer from 0 to 6, preferably 0, 1 or 2, and more preferably 0.

7. The compound of formula (I) or the mesomer, racemate, enantiomer, diastereomer thereof, or mixture thereof, or the prodrug thereof, or the pharmaceutically acceptable salt thereof according to any one of claims 1 to 6,
wherein,
each R⁸ can be identical or different and are each independently selected from the group consisting of halogen, alkyl, haloalkyl, alkoxy and haloalkoxy;
q is an integer from 0 to 4; and preferably q is 0 or 1.

8. The compound of formula (I) or the mesomer, racemate, enantiomer, diastereomer thereof, or mixture thereof, or the prodrug thereof, or the pharmaceutically acceptable salt thereof according to any one of claims 1 to 7, wherein the compound is selected from the group consisting of:
4-(5-((1R,3r,5S)-3-((5-cyclopropyl-3-(2,6-dichlorophenyl)-isoxazol-4-yl)methoxy) -8-azabicyclo[3.2.1]octan-8-yl)-1,2,4-oxadiazol-3-yl)benzoic acid;
2-chloro-4-(5-((1R,3r,5S)-3-((5-cyclopropyl-3-(2,6-dichlorophenyl)isoxazol-4-yl) methoxy)-8-azabicyclo[3.2.1]octan-8-yl)-1,2,4-oxadiazol-3-yl)benzoic acid;
6-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)pyridine-2-carboxylic acid;
5-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)thiophene-2-carboxylic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)-3-methylbenzoic acid;
3-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)benzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,2,4-oxadiazol-3-yl)-2-methylbenzoic acid;
4-(5-((1R,3r,5S)-3-((5-cyclopropyl-3-(2,6-dichlorophenyl)isoxazol-4-yl)methoxy)-8-azabicyclo[3.2.1]octan-8-yl)-1,3,4-oxadiazol-2-yl)benzoic acid;
2-chloro-4-(5-((1R,3r,5S)-3-((5-cyclopropyl-3-(2,6-dichlorophenyl)isoxazol-4-yl) methoxy)-8-azabicyclo[3.2.1]octan-8-yl)-1,3,4-oxadiazol-2-yl)benzoic acid;
6-(5-((1R,3r,5S)-3-((5-cyclopropyl-3-(2,6-dichlorophenyl)isoxazol-4-yl)methoxy)-8-azabicyclo[3.2.1]octan-8-yl)-1,3,4-oxadiazol-2-yl)pyridine-2-carboxylic acid;
5-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)thiophene-2-carboxylic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-2-fluorobenzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-2-methoxybenzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-3-methylbenzoic acid;
3-(5-((1R,3r,5 S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)benzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-3-fluorobenzoic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)-2-methylbenzoic acid;
5-(5-((1R,3r,5S)-3-((5-cyclopropyl-3-(2,6-dichlorophenyl)isoxazol-4-yl)methoxy)-8-azabicyclo[3.2.1]octan-8-yl)-1,3,4-oxadiazol-2-yl)pyridine-carboxylic acid;
6-(5-((1R,3r,5 S)-3-((5-cyclopropyl-3-(2,6-dichlorophenyl)isoxazol-4-yl)methoxy)-8-azabicyclo[3.2.1]octan-8-yl)-1,3,4-oxadiazol-2-yl)nicotinic acid;
5-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)thiophene-3-carboxylic acid;
4-(5-((1R,3r,5S)-(3-((5-cyclopropyl-3-(2,6-dichlorophenyl)isoxazol-4-yl)methoxy) -8-azabicyclo[3.2.1]octan-8-yl)-1,3,4-oxadiazol-2-yl)thiophene-2-carboxylic acid;
4-(5-((1R,3r,5S)-3-((5-cyclopropyl-3-(2-(trifluoromethoxy)phenyl)isoxazol-4-yl)m ethoxy)-8-azabicyclo[3.2.1]octan-8-yl)-1,3,4-oxadiazol-2-yl)benzoic acid;
2-chloro-4-(5-((1R,3r,5S)-3-((5-cyclopropyl-3-(2-(trifluoromethoxy)phenyl)isoxaz ol-4-yl)methoxy)-8-azabicyclo[3.2.1]octan-8-yl)-1,3,4-oxadiazol-2-yl)benzoic acid;
6-(5-((1R,3r,5S)-3-((5-cyclopropyl-3-(2-(trifluoromethoxy)phenyl)isoxazol-4-yl)m ethoxy)-8-azabicyclo[3.2.1]octan-8-yl)-1,3,4-oxadiazol-2-yl)pyridine-2-carboxylic acid;
5-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)thiophene-2-carboxylic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)-2-fluorobenzoic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)-3-methylbenzoic acid;
3-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)benzoic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)-3-fluorobenzoic acid;
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)-2-methylbenzoic acid;
5-(5-((1R,3r,5S)-3-((5-cyclopropyl-3-(2-(trifluoromethoxy)phenyl)isoxazol-4-yl)m ethoxy)-8-azabicyclo[3.2.1]octan-8-yl)-1,3,4-oxadiazol-2-yl)pyridine-carboxylic acid;
6-(5-((1R,3r,5S)-3-((5-cyclopropyl-3-(2-(trifluoromethoxy)phenyl)isoxazol-4-yl)m ethoxy)-8-azabicyclo[3.2.1]octan-8-yl)-1,3,4-oxadiazol-2-yl)nicotinic acid;
5-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)thiophene-3-carboxylic acid; and
4-(5-((1R,3r,5S)-(3-((5-cyclopropyl-3-(2-(trifluoromethoxy)phenyl)isoxazol-4-yl) methoxy)-8-azabicyclo[3.2.1]octan-8-yl)-1,3,4-oxadiazol-2-yl)thiophene-2-carboxylic acid.

9. A method for preparing the compound of formula (I) or the mesomer, racemate, enantiomer, diastereomer thereof, or mixture thereof, or the prodrug thereof, or the pharmaceutically acceptable salt thereof according to any one of claims 1 to 8, comprising the following step of: 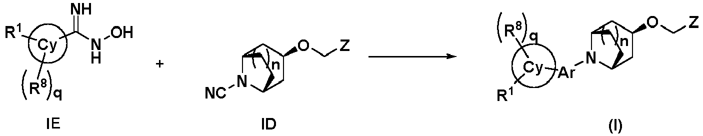 subjecting compound IE and compound ID to a cyclization reaction in the presence of a condensing agent to obtain the compound of formula (I), wherein the condensing agent is preferably zinc chloride;
wherein Ar is 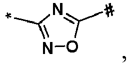 wherein ^{∗} represents the site connected to Cy, and # represents the site connected to the N of the bridged ring,
Z, n, Cy, R¹, R⁸ and q are as defined in claim 1.

10. A method for preparing the compound of formula (I) or the mesomer, racemate, enantiomer, diastereomer thereof, or mixture thereof, or the prodrug thereof, or the pharmaceutically acceptable salt thereof according to any one of claims 1 to 8, comprising the following step of: 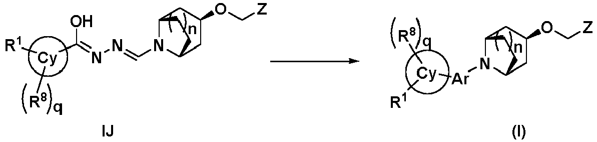 subjecting compound IJ to a cyclization reaction in the presence of an oxidizing agent to obtain the compound of formula (I), wherein the oxidizing agent is preferably iodobenzene diacetate;
when Ar is 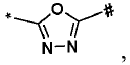 wherein ^{∗} represents the site connected to Cy, and # represents the site connected to the N of the bridged ring,
Z, n, Cy, R¹, R⁸ and q are as defined in claim 1.

11. A pharmaceutical composition comprising the compound of formula (I) or the mesomer, racemate, enantiomer, diastereomer thereof, or mixture thereof, or the prodrug thereof, or the pharmaceutically acceptable salt thereof according to any one of claims 1 to 8, and a pharmaceutically acceptable carrier.

12. Use of the compound of formula (I) or the mesomer, racemate, enantiomer, diastereomer thereof, or mixture thereof, or the prodrug thereof, or the pharmaceutically acceptable salt thereof according to any one of claims 1 to 8, or the pharmaceutical composition according to claim 11 in the preparation of a FXR agonist.

13. Use of the compound of formula (I) or the mesomer, racemate, enantiomer, diastereomer thereof, or mixture thereof, or the prodrug thereof, or the pharmaceutically acceptable salt thereof according to any one of claims 1 to 8, or the pharmaceutical composition according to claim 11 in the preparation of a medicament for preventing and/or treating a disease related to FXR activity.

14. The use according to claim 13, wherein the disease related to FXR activity is selected from the group consisting of chronic intrahepatic cholestasis or extrahepatic cholestasis, or liver fibrosis caused by chronic cholestasis or acute intrahepatic cholestasis; liver obstructive or chronic inflammation; liver cirrhosis; hepatic steatosis and related syndromes, cholestasis or fibrosis associated with alcohol-induced cirrhosis or viral hepatitis; liver failure or liver ischemia after liver resection; chemotherapy related to steatohepatitis; acute liver failure; inflammatory bowel disease; lipid and lipid protein disorders; diabetes and clinical complications of diabetes, including diabetic nephropathy, diabetic neuropathy, diabetic retinopathy and other clinical manifestations; lipids, especially triglyceride accumulation, and diseases and disorders caused by chronic fat and fibrosis due to triglyceride accumulation, such as non-alcoholic fatty liver or non-alcoholic steatohepatitis; obesity or metabolic syndrome, such as dyslipidemia, diabetes, and comorbidities with abnormally high body mass index; acute myocardial infarction, acute stroke or thrombosis as the end point of chronic obstructive atherosclerosis; non-malignant hyperproliferative diseases and malignant hyperproliferative diseases, especially hepatocellular carcinoma, colonic adenoma and polyposis, colon adenocarcinoma, breast cancer, pancreatic cancer, Bart's esophagus cancer and other forms of gastrointestinal and liver neoplastic diseases.
